# EUROPEAN PATENT SPECIFICATION

(11) **EP 2 430 036 B1**
(45) Date of publication and mention of the grant of the patent: **30.07.2014**
(21) Application number: 10736593.4
(22) Date of filing: 12.05.2010
(51) Int. Cl.: C07H 19/167, A61K 31/7076

(54) **SUBSTITUTED 6-(BENZYLAMINO) PURINE RIBOSIDE DERIVATIVES, USE THEREOF AND COMPOSITIONS CONTAINING THESE DERIVATIVES**
SUBSTITUIERTE 6-(BENZYLAMINO)PURINRIBOSIDDERIVATE, DEREN VERWENDUNG UND ZUSAMMENSETZUNGEN, DIE DIESE DERIVATE ENTHALTEN
DÉRIVÉS DE RIBOSIDE DE 6-BENZYLAMINOPURINE SUBSTITUÉE, LEURS UTILISATIONS ET COMPOSITIONS CONTENANT CES DÉRIVÉS

(30) Priority: 14.05.2009 CZ 200900298 U
(43) Date of publication of application: 21.03.2012
(73) Proprietor: Univerzita Palackého v Olomouci, 771 47 Olomouc (CZ); Biopatterns s.r.o., 779 00 Olomouc (CZ)
(72) Inventor: SZUCOVA, Lucie, 779 00 Olomouc (CZ); KRYSTOF, Vladimir, 779 00 Olomouc (CZ); ZATLOUKAL, Marek, 787 01 Sumperk (CZ); DOLEZAL, Karel, 783 65 Hlubocky (CZ); STRNAD, Miroslav, 779 00 Olomouc (CZ); SPICHAL, Lukas, 779 00 Olomouc (CZ)
(74) Representative: Gabrielova, Marta
(86) International application number: PCT/CZ2010/000061
(87) International publication number: WO 2010/130233

(56) References cited:
- WO-A1-93/23418
- WO-A1-2006/125190
- WO-A2-2004/058791
- DE-A1- 1 670 175
- DE-A1- 1 670 265
- DE-A1- 2 055 160
- DE-A1- 2 524 284
- US-A1- 2006 166 248
- COSYN LIESBET ET AL: "2-Triazole-Substituted Adenosines: A New Class of Selective A3 Adenosine Receptor Agonists, Partial Agonists, and Antagonists" JOURNAL OF MEDICINAL CHEMISTRY, AMERICAN CHEMICAL SOCIETY, WASHINGTON, US LNKD- DOI:10.1021/JM0608208, vol. 49, no. 25, 14 December 2006 (2006-12-14), pages 7373-7383, XP009132900 ISSN: 0022-2623 [retrieved on 2006-11-15]
- BRESSI J C ET AL: "ADENOSINE ANALOGUES AS INHIBITORS OF TRYPANOSOMA BRUCEI PHOSPHOGLYCERATE KINASE: ELUCIDATION OF A NOVEL BINDING MODE FOR A 2-AMINO-N6-SUBSTITUTED ADENOSINE" JOURNAL OF MEDICINAL CHEMISTRY, AMERICAN CHEMICAL SOCIETY, WASHINGTON, US LNKD- DOI:10.1021/JM000287A, vol. 43, no. 22, 1 January 2000 (2000-01-01), pages 4135-4150, XP000999137 ISSN: 0022-2623

## Description

### Technical Field

The invention relates to 2-substited-6-(substituted benzylamino)purine riboside derivatives, to their use as antiapoptotic and anti-inflammatory factors of neutrophils and to pharmaceutical preparations containing these derivatives.

### Background Art

Neutrophil granulocytes, generally referred to as *neutrophils,* are the most abundant type of white blood cells and form an integral part of the immune system. Their name derives from staining characteristics on hematoxylin and eosin (H&E) histological preparations. Whereas basophilic cellular components stain dark blue and eosinophilic components stain bright red, neutrophilic components stain a neutral pink. These phagocytes are normally found in the blood stream. However, during the acute phase of inflammation, particularly as a result of bacterial infection, neutrophils leave the vasculature and migrate toward the site of inflammation in a process called chemotaxis. They are the predominant cells in pus, accounting for its whitish/yellowish appearance. Neutrophils react within an hour of tissue injury and are the hallmark of acute inflammation (Cohen, Stephen. Burns, Richard C. Pathways of the Pulp, 8th Edition. St. Louis: Mosby, Inc. 2002. p. 465). The average halflife of a non-activated neutrophil in the circulation is about 4-10 hours. Upon activation, they marginate (position themselves adjacent to the blood vessel endothelium), undergo selectin dependent capture followed by integrin dependent adhesion after which they migrate into tissues, where they survive for 1-2 days. Neutrophils are much more numerous than the longer-lived monocytes/macrophages. The first phagocyte a pathogen (disease-causing microorganism) is likely to encounter is a neutrophil. Some experts feel that the short lifetime of neutrophils is an evolutionary adaptation to minimize propagation of those pathogens that parasitize phagocytes. The more time such parasites spend outside a host cell, the more likely they will be destroyed by some component of the body's defenses. However, because neutrophil antimicrobial products can also damage host tissues, other authorities feel that their short life is an adaptation to limit damage to the host during inflammation. Neutrophils are phagocytes, capable of ingesting microorganisms or particles. They can internalise and kill many microbes, each phagocytic event resulting in the formation of a phagosome into which reactive oxygen species and hydrolytic enzymes are secreted. The consumption of oxygen during the generation of reactive oxygen species has been termed the "respiratory burst," although it actually has nothing to do with respiration or energy production. The respiratory burst involves the activation of the enzyme NADPH oxidase, which produces large quantities of superoxide, a reactive oxygen species. Superoxide dismutates, spontaneously or through catalysis via the enzyme catalase, to hydrogen peroxide, which is then converted to hypochlorous acid (HOCl, also known as chlorine bleach) by the green heme enzyme myeloperoxidase. It is thought that the bactericidal properties of HOCl are enough to kill bacteria phagocytosed by the neutrophil, but this has not been proven conclusively.

Low neutrophil counts are termed "neutropenia". This can be congenital (genetic disorder) or it can develop later, as in the case of aplastic anemia or some kinds of leukemia. It can also be a side-effect of medication, most prominently chemotherapy. Neutropenia predisposes heavily for infection. Finally, neutropenia can be the result of colonization by intracellular neutrophilic parasites. Functional disorders of neutrophils are often hereditary. The term neutropenia is defined as a decrease in circulating neutrophils to less than 1.5 x 10⁹ / 1. Neutropenia may be classified according to the mechanism of its formation into several classes. It may be the neutropenia caused by a reduced production of neutrophils, disturbances or changes in the release of endothelial cells or tissue reservoirs or in increased disappearance and destruction of neutrophils. The clinical manifestation of neutropenia is the increased possibility of infection. The severity of these infections depends on the quantitative and in some cases qualitative defects of neutrophils, which in the rheumatic diseases are often caused by circulating neutrophil antibodies at different levels of development. Neutropenia may occur in patients with systemic autoimmune diseases - lupus erythematosus, Felty syndrome, autoimmune hemolytic anemia or immune thrombocytopenia, Sjögren's syndrome. The disease is caused by autoantibodies against neutrophil specific antigens. These are disorders of phagocytosis or respiratory burst (such as chronic granulomatosis, a rare disorder of immunity and lack of myeloperoxidase). In the absence of alpha 1-antitrypsin is elastase, an important enzyme of neutrophils, poorly inhibited by alpha 1-antitrypsin, leading to excessive tissue damage in case of infection - particularly emphysema. Hereditary familial Mediterranean fever (FMF) is an autosomal recessive disease affecting mainly population around the Mediterranean Sea. Patients suffering from periodic fever accompanied with serositis and arthritis, a late complication is the development of amyloidosis. FMF is caused by mutations in the gene encoding the protein pyrin / marenostrin (16 chromosome) expressed in neutrophils and in particular reflecting the cyclic fluctuations of neutrophil leukocytes in peripheral blood. Pyrin is phylogenetically a very old protein with a nonspecific anti-inflammatory effect. It occurs exclusively in the peripheral polymorphonuclears and mutant form is unable to inhibit the normal inflammatory undesirable impulses, which is the essence of febrile attacks of FMF. Another disease caused by neutrophils is vasculitis. Vasculitides are a heterogeneous group of diseases characterized by inflammation, necrosis of blood vessels, which leads to failure of blood supply to the area served by the relevant vessels. In the pathogenesis, there are several mechanisms for: (a) inflammatory reaction triggered by immune complexes-in tissue deposits of immune complexes and complement component C3, (b) autoantibodies against neutrophil lyzosomal enzymes (so-called ANCA - Anti neutrophil Cytoplasmic Antibodies) - they stimulate neutrophils to produce oxygen radicals and to the secretion of lysosomal enzymes that causes the damage to surrounding tissue.

The cytokinins (CK), which represent one of the major groups of plant hormones, were discovered in the search for factors that stimulate plant cell division. In the mid 1950's, Miller and Skoog described the first cytokinin, kinetin (K) (Miller et al. et al., J. Am. Chem. Soc. 77:1392-1392, 1955). Later, zeatin (Z) was identified as the first naturally occurring cytokinin in immature maize endosperm (Letham D.S. Life Sci. 8:569-573, 1963), and turned out to be the abundant cytokinin in coconut milk. Although cytokinins regulate many other important physiological processes, the control of cell division (*cytokinesis*) is crucial and is considered diagnostic for this class of phytohormones; hence the generic name, *cytokinins.* These compounds have the ability to induce cell division via involvement in the cell cycle stages (cyclins induction in G1 phase). Regulators that play important roles in the differentiation and development of plants and invertebrates may also affect the differentiation of normal and malignant cells in man and might be clinically useful for treating some cancers (Mlejnek and Kuglik, 2000 J. Cell. Biochem. 77:6-17 2000).

Extracellular purine ribosides have been identified as effective modulators of cell growth and inductors of differentiation in numerous cell types (Rathbone et al., Med. Hypotheses 37: 232-240: 1992). Recent evidence suggests that purine ribosides take part in the signal transduction of many pathological processes including cell death. Their effect is likely to be mediated through activation of membrane-bound purine riboside receptors (Franceschi et al., Drug Dev. Res. 39: 442-449, 1996). In general, two distinct cytotoxic mechanisms are considered, intracellular, when purine ribosides may become cytotoxic after their intracellular phosphorylation (Cottam et al. J. Med. Chem. 36: 3424-3430, 1993) and extracellular, when cell death is mediated via extracellular adenosine receptors (Kohno et al., Biochem. Biophys. Res. Commun. 221: 849-849, 1996). Ribosides are one of the forms in which purine ribosides naturally occur. It has been discovered that these cytokinin derivatives, that act as hormones and control many processes in plants, are also very effective inhibitors of cancer cell proliferation and very potent inducers of apoptosis with typical morphological and biochemical hallmarks (Ishii et al., Biochim. Biophys. Acta-Mol. Cell Res. 1643: 11-24, 2003). When their mechanism of action was investigated in more detail, it was discovered that as in plants, cytokinin nucleosides have similar differentiation-inducing activity in several human leukemia cell lines, as in the case of their redifferentiation-inducing activities in plants (Ishii et al., Cell Growth & Differentiation 13: 19-26,2002).

Cytokinin bases, such as kinetin, isopentenyladenine, and 6-benzylaminopurine riboside are very effective in inducing morphological changes in the cells into mature granulocytes. On the other hand, cytokinin ribosides, such as kinetin riboside, isopentenyladeriosine, and 6-benzylaminopurine riboside are the most potent for growth inhibition and apoptosis. Cytokinin ribosides also greatly reduce the intracellular ATP content and induce mitochondrial disruption, whereas free bases protect against mitochondrial disruption and apoptosis in leukemia cells (Ishii et al., Cell Growth & Differentiation 13: 19-26, 2002). These results suggest why both cytokinins and cytokinin ribosides can induce granulocytic differentiation of HL-60 cells but cytokinin ribosides also induce apoptosis prior to the differentiation process. Although the effects of cytokinins on plant development are well-known, the mechanism of cytokinin action is still not completely understood. In plants, cytokinin binding proteins and cytokinin receptors have been isolated and shown to be involved in the cytokinin-signaling pathway. However, this signaling pathway seems to be unlikely in human cancer cells. The findings that adenosine receptor A1 and A2 inhibitors affect the action of cytokinins on HL-60 cells (Ishii et al., Biochim. Biophys. Acta-Mol. Cell Res. 1643: 11-24, 2003) suggested that the mechanism of cytokinin-induced differentiation could be closely related to a metabolic process that require intracellular phosphorylation of benzylaminopurine riboside (nucleotide formation) by adenosine kinase or its related enzymes and that this process is necessary for the manifestation of its cytotoxicity (Mlejnek and Kuglik, J. Cell. Biochem. 77:6-17 2000). Moreover, it has been reported that caspases might be critically implicated in the apoptotic process (Mlejnek and Procházka, Planta 215, 158-166: 2002). In 2005 Mlejnek & Prochazka demonstrated that adenine as well as adenosine derivatives were phosphorylated within cells to the monophosphate level. While N⁶-substituted derivatives of adenosine were phosphorylated by adenosine kinase and corresponding mononucleotides were produced in large quantities, N⁶-substituted derivatives of adenine were converted into the corresponding mononucleotides via the phosphoribosyl transferase pathway, which yielded 50-100 times lower amounts of the mononucleotides than the adenosine kinase pathway. Accordingly, N⁶-substituted derivatives of adenine were relatively inefficient inductors of apoptosis at the concentrations applied. Inhibitors of adenosine kinase that abrogated the formation of monophosphates from N⁶-substituted derivatives of adenosine completely prevented cells from going into apoptosis. These results consistently support the idea that pro-apoptotic effects of N⁶-substituted derivatives of adenosine are related to their intracellular conversion into corresponding mononucleotides which eventually trigger apoptosis when accumulated beyond a certain level. Intracellular accumulation of mononucleotides derived from the corresponding N⁶-substituted derivatives of adenosine led to a rapid decrease in ATP production and consequently to apoptosis induction (Mlejnek et al., Plant Sci. 168: 389-395, 2005).

Nevertheless, the mechanism of action, the apoptosis pathways and the signalling intermediates of cytokinin-induced programmed cell death have not yet been fully described and remain to be elucidated.

We have now discovered neutrophil growth factors based on 2-substituted-6-(substituted benzylamino)purine ribosides. The most promising substituents at C2 of the purine ring are halogen, in particular chloro, fluoro, iodo, and alkylamino groups which modulate activity of neutrophils in direction to specific antiapoptoic affects. Additionally, the prepared compounds showed interesting *in vivo* effect on inflammation of model mice.

It is theretofore one object of this invention to provide growth-regulatory, differentiating and antiapoptotic cytokinin analogues having high selectivity and efficiency index, i.e. that are nontoxic yet more efficacious than cytokinin analogues known heretofore.

It is another object of the invention to provide anti-inflammatory cytokinin analogues having a high selectivity and being nontoxic.

### Disclosure of the Invention

Object of this invention are substituted 2-substituted-6-(substituted benzylamino)purine riboside derivatives of the general formula I, 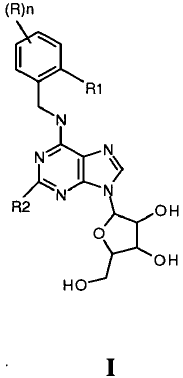 wherein
**(R)ₙ** represents 0 to 4 substituents (**n** is 0-4), which can be the same or different, the substituents being selected from the group comprising alkyl, alkoxy, amino, halogen, hydroxyl, nitro, mercapto and alkylmercapto, and
**R1** is selected from the group consisting of halogen, hydroxy, amino, alkoxy, mercapto, alkylmercapto, alkyl, and
**R2** is selected from the group consisting of halogen, amino, azido, alkoxy, mercapto, alkylmercapto, alkyl, alkenyl, alkynyl, -NHNH₂, -NHOH, -NHCONH₂, guanylo (-NH-C(NH)NH₂), and
**R2** is **R2'-N(R3)-** wherein
**R2'** is selected from the group consisting of alkyl, alkenyl, cycloalkyl, phenyl and benzyl, wherein each of the groups can optionally be substituted by one or more substituents selected from the group comprising amino, halogen, hydroxy, alkoxy, mercapto and alkylmercapto,
**R3** is selected from hydrogen and alkyl, said alkyl being unsubstituted or substituted by one or more substituents selected from the group comprising amino, halogen, hydroxy, alkoxy, mercapto and alkylmercapto,
and the pharmaceutically acceptable salts thereof with alkali metals, ammonium or amines, or their addition salts with acids,
for use in therapeutic suppression of apoptosis and the inflammatory activity of neutrophils.

As used herein, and unless modified by the immediate context, the generic substituent groups have the following meanings:
amino denotes the group -NH₂,
azido denotes the group -N₃,
halogen is selected from fluorine, bromine, chlorine and iodine atom,
mercapto denotes the group -SH,
hydroxy denotes the group -OH,
alkyl denotes a branched or unbranched alkyl chain containing 1 to 8 carbon atoms, preferably selected from the group comprising methyl, ethyl, propyl, isopropyl, butyl, isobutyl, pentyl, isopentyl, hexyl, isohexyl,
alkenyl denotes a branched or unbranched alkenyl chain containing 2 to 7 carbon atoms, preferably selected from the group comprising vinyl, allyl, 1-propenyl, 1-methylethenyl, but-1 to 3-enyl, pent-1 to 4-enyl, isopentenyl, hex-1 to 5-enyl, hept-1 to 6-enyl,
alkynyl denotes a branched or unbranched alkynyl chain containing 2 to 7 carbon atoms,
cycloalkyl denotes a monocyclic or polycyclic alkyl group containing 3 to 8 carbon atoms,
alkoxy denotes -O-Rₐ, where Rₐ is alkyl or cycloalkyl,
alkylmercapto denotes the group -SR_{b}, where R_{b} is alkyl or cycloalkyl.

In the preferred embodiment, the substituted 6-(benzylamino)purine riboside derivatives of the general formula I are selected from the group comprising 2-(amino, chloro, fluoro, mercapto, methyl, C₁-C₆-alkylmercapto, C₁-C₆-alkylamino, hydroxy(C₁-C₆)alkylamino, amino(C₁-C₆)alkylamino)-6-(2-hydroxy-3-methoxybenzylamino)purine riboside, 2-(amino, chloro, fluoro, mercapto, methyl, C₁-C₆-alkylmercapto, C₁-C₆-alkylamino, hydroxy(C₁-C₆)alkylamino, amino(C₁-C₆)alkylamino)-6-(2-hydroxy-4-methoxybenzylamino)purine riboside, 2-(amino, chloro, fluoro, mercapto, methyl, C₁-C₆-alkylmercapto, C₁-C₆-alkylamino, hydroxy(C₁-C₆)alkylamino, amino(C₁-C₆)alkylamino)-6-(2-hydroxy-5-methoxybenzylamino)-purine riboside, 2-(amino, chloro, fluoro , mercapto, methyl, C₁-C₆-alkylmercapto, C₁-C₆-alkylamino, hydroxy(C₁-C₆)alkylamino, amino(C₁-C₆)alkylamino)-6-(2-hydroxy-6-methoxybenzylamino)purine riboside, 2-(amino, chloro, fluoro , mercapto, C₁-C₆-alkylmercapto, C₁-C₆-alkylamino, hydroxy(C₁-C₆)alkylamino, amino (C₁-C₆)alkylamino)-6-(2,3-dihydroxy-4-methoxybenzylamino)purine riboside, 2-(amino, chloro, fluoro, , methyl, mercapto, C₁-C₆-alkylmercapto, C₁-C₆-alkylamino, hydroxy(C₁-C₆)alkylamino, amino(C₁-C₆)alkylamino)-6-(2,5-dihydroxy-4-methoxy-benzylamino)purine riboside, 2-(amino, chloro, fluoro , methyl, mercapto, C₁-C₆-alkylmercapto, C₁-C₆-alkylamino, hydroxy(C₁-C₆)alkylamino, amino(C₁-C₆)alkylamino)-6-(2-hydroxy-3-methylbenzylamino)purine riboside, 2-(amino, chloro, fluoro, mercapto, methyl, C₁-C₆-alkylmercapto, C₁-C₆-alkylamino, hydroxy(C₁-C₆)alkylamino, amino(C₁-C₆)alkylamino)-6-(2-hydroxy-4-methylbenzylamino)purine riboside, 2-(amino, chloro, fluoro, mercapto, methyl, C₁-C₆-alkylmercapto, C₁-C₆-alkylamino, hydroxy(C₁-C₆)alkylamino, amino(C₁-C₆)alkylamino)-6-(2-hydroxy-5-methylbenzylamino)purine riboside, 2-(amino, chloro, fluoro, mercapto, methyl, C₁-C₆-alkylmercapto, C₁-C₆-alkylamino, hydroxy(C₁-C₆)alkylamino, amino(C₁-C₆)alkylamino)-6-(2-hydroxy-6-methylbenzylamino)purine riboside, 2-(amino, chloro, fluoro, mercapto, methyl, C₁-C₆-alkylmercapto, C₁-C₆-alkylamino, hydroxy(C₁-C₆)alkylamino, amino(C₁-C₆)alkylamino)-6-(2-hydroxy-3-chlorobenzylamino)purine riboside, 2-(amino, chloro, fluoro, mercapto, methyl, C₁-C₆-alkylmercapto, C₁-C₆-alkylamino, hydroxy(C₁-C₆)alkylamino, amino(C₁-C₆)alkylamino)-6-(2-hydroxy-4-chlorobenzylamino)purine riboside, 2-(amino, chloro, fluoro, mercapto, methyl, C₁-C₆-alkylmercapto, C₁-C₆-alkylamino, hydroxy(C₁-C₆)alkylamino, amino(C₁-C₆)alkylamino)-6-(2-hydroxy-5-chlorobenzylamino)purine riboside, 2-(amino, chloro, fluoro, mercapto, methyl, C₁-C₆-alkylmercapto, C₁-C₆-alkylamino, hydroxy(C₁-C₆)alkylamino, amino(C₁-C₆)alkylamino)-6-(2-hydroxy-6-chlorobenzylamino)purine riboside, 2-(amino, chloro, fluoro, mercapto, methyl, C₁-C₆-alkylmercapto, C₁-C₆-alkylamino, hydroxy(C₁-C₆)alkylamino, amino(C₁-C₆)alkylamino)-6-(2-hydroxy-3-fluorobenzylamino)purine riboside, 2-(amino, chloro, fluoro , mercapto, methyl, C₁-C₆-alkylmercapto, C₁-C₆-alkylamino, hydroxy(C₁-C₆)alkylamino, amino(C₁-C₆)alkylamino)-6-(2-hydroxy-5-fluorobenzylamino)purine riboside, 2-(amino, chloro, fluoro, mercapto, methyl, C₁-C₆-alkylmercapto, C₁-C₆-alkylamino, hydroxy(C₁-C₆)alkylamino, amino(C₁-C₆)alkylamino)-6-(2-hydroxy-3-bromobenzylamino)purine riboside, 2-(amino, chloro, fluoro, mercapto, methyl, C₁-C₆-alkylmercapto, C₁-C₆-alkylamino, hydroxy(C₁-C₆)alkylamino, amino(C₁-C₆)alkylamino)-6-(2-hydroxy-3-iodobenzylamino)purine riboside, 2-(amino, chloro, fluoro, mercapto, methyl, C₁-C₆-alkylmercapto, C₁-C₆-alkylamino, hydroxy(C₁-C₆)alkylamino, amino(C₁-C₆)alkylamino)-6-(2,3-dihydroxy-4-methylbenzylamino) purine riboside, 2-(amino, chloro, fluoro, mercapto, methyl, C₁-C₆-alkylmercapto, C₁-C₆-alkylamino, hydroxy(C₁-C₆)alkylamino, amino(C₁-C₆)alkylamino)-6-(2,5-dihydroxy-4-methylbenzylamino)purine riboside, 2-(amino, chloro, fluoro, mercapto, methyl, C₁-C₆-alkylmercapto, C₁-C₆-alkylamino, hydroxy(C₁-C₆)alkylamino, amino(C₁-C₆)alkylamino)-6-(2,3-dihydroxy-4-chlorobenzylamino)purine riboside, 2-(amino, chloro, fluoro, mercapto, methyl, C₁-C₆-alkylmercapto, C₁-C₆-alkylamino, hydroxy(C₁-C₆)alkylamino, amino(C₁-C₆)alkylamino)-6-(2,5-dihydroxy-4-chlorobenzylamino)purine riboside, 2-(amino, chloro, fluoro, mercapto, methyl, C₁-C₆-alkylmercapto, C₁-C₆-alkylamino, hydroxy(C₁-C₆)alkylamino, amino(C₁-C₆)alkylamino)-6-(2,6-dihydroxy-4-chlorobenzylamino) purine riboside, 2-(amino, chloro, fluoro, mercapto, methyl, C₁-C₆-alkylmercapto, C₁-C₆-alkylamino, hydroxy(C₁-C₆)alkylamino, amino(C₁-C₆)alkylamino)-6-(2-hydroxy-3-aminobenzylamino)purine riboside, 2-(amino, chloro, fluoro, mercapto, methyl, C₁-C₆-alkylmercapto, C₁-C₆-alkylamino, hydroxy(C₁-C₆)alkylamino, amino(C₁-C₆)alkylamino)-6-(2-hydroxy-4-aminobenzylamino)purine riboside, 2-(amino, chloro, fluoro, methyl, mercapto, C₁-C₆-alkylmercapto, C₁-C₆-alkylamino, hydroxy(C₁-C₆)alkylamino, amino(C₁-C₆)alkylamino)-6-(2-hydroxy-5-aminobenzylamino)purine riboside, 2-(amino, chloro, methyl,, methyl, mercapto, C₁-C₆-alkylmercapto, C₁-C₆-alkylamino, hydroxy(C₁-C₆)alkylamino, amino(C₁-C₆)alkylamino)-6-(2-hydroxy-5-mercaptobenzylamino)purine riboside, 2-(amino, chloro, fluoro, methyl, mercapto, C₁-C₆-alkylmercapto, C₁-C₆-alkylamino, hydroxy(C₁-C₆)alkylamino, amino(C₁-C₆)alkylamino)-6-(2-amino-3-methoxybenzylamino)purine riboside, 2-(amino, chloro, fluoro, methyl,mercapto, C₁-C₆-alkylmercapto, C₁-C₆-alkylamino, hydroxy(C₁-C₆)alkylamino, amino(C₁-C₆)alkylamino)-6-(2-amino-4-methoxybenzylamino)purine riboside, 2-(amino, chloro, fluoro, methyl,mercapto, C₁-C₆-alkylmercapto, C₁-C₆-alkylamino, hydroxy(C₁-C₆)alkylamino, amino(C₁-C₆)alkylamino)-6-(2-amino-5-methoxybenzylamino)purine riboside, 2-(amino, chloro, fluoro, methyl, mercapto, C₁-C₆-alkylmercapto, C₁-C₆-alkylamino, hydroxy(C₁-C₆)alkylamino, amino(C₁-C₆)alkylamino)-6-(2-amino-3-methylbenzylamino)purine riboside, 2-(amino, chloro, fluoro, methyl,, mercapto, C₁-C₆-alkylmercapto, C₁-C₆-alkylamino, hydroxy(C₁-C₆)alkylamino, amino(C₁-C₆)alkylamino)-6-(2-amino-5-methylbenzylamino)purine riboside, 2-(amino, chloro, fluoro, methyl, mercapto, C₁-C₆-alkylmercapto)-6-(2-amino-3-chlorobenzylamino)purine riboside, 2-(amino, chloro, fluoro, methyl, mercapto, C₁-C₆-alkylmercapto, C₁-C₆-alkylamino, hydroxy(C₁-C₆)alkylamino, amino(C₁-C₆)alkylamino)-6-(2-amino-4-chlorobenzylamino)purine riboside, 2-(amino, chloro, fluoro, methyl, mercapto, C₁-C₆-alkylmercapto, C₁-C₆-alkylamino, hydroxy(C₁-C₆)alkylamino, amino(C₁-C₆)alkylamino)-6-(2-amino-5-chlorobenzylamino)purine riboside, 2-(amino, chloro, fluoro, methyl, mercapto, C₁-C₆-alkylmercapto, C₁-C₆-alkylamino, hydroxy(C₁-C₆)alkylamino, amino(C₁-C₆)alkylamino)-6-(2-amino-6-chlorobenzylamino)purine riboside, 2-(amino, chloro, fluoro, methyl, mercapto, C₁-C₆-alkylmercapto, C₁-C₆-alkylamino, hydroxy(C₁-C₆)alkylamino, amino(C₁-C₆)alkylamino)-6-(2-amino-3-fluorobenzylamino)purine riboside, 2-(amino, chloro, fluoro, methyl,, mercapto, C₁-C₆-alkylmercapto, C₁-C₆-alkylamino, hydroxy(C₁-C₆)alkylamino, amino(C₁-C₆)alkylamino)-6-(2-amino-5-fluorobenzylamino)purine riboside, 2-(amino, chloro, fluoro, methyl, mercapto, C₁-C₆-alkylmercapto, C₁-C₆-alkylamino, hydroxy(C₁-C₆)alkylamino, amino(C₁-C₆)alkylamino)-6-(2-amino-3-bromobenzylamino)purine riboside, 2-(amino, chloro, fluoro, methyl,, mercapto, C₁-C₆-alkylmercapto, C₁-C₆-alkylamino, hydroxy(C₁-C₆)alkylamino, amino(C₁-C₆)alkylamino)-6-(2,3-diaminobenzylamino)purine riboside, 2-(amino, chloro, fluoro, methyl, mercapto, C₁-C₆-alkylmercapto, C₁-C₆-alkylamino, hydroxy(C₁-C₆)alkylamino, amino(C₁-C₆)alkylamino)-6-(2,5-diaminobenzylamino)purine riboside, 2-(amino, chloro, fluoro, methyl,, mercapto, C₁-C₆-alkylmercapto, C₁-C₆-alkylamino, hydroxy(C₁-C₆)alkylamino, amino(C₁-C₆)alkylamino)-6-(2,6-diamino-3-chlorobenzylamino)purine riboside, 2-(amino, chloro, fluoro, methyl, mercapto, C₁-C₆-alkylmercapto, C₁-C₆-alkylamino, hydroxy(C₁-C₆)alkylamino, amino(C₁-C₆)alkylamino)-6-(2,6-diamino-4-chlorobenzylamino)purine riboside, 2-(amino, chloro, fluoro, methyl, mercapto, C₁-C₆-alkylmercapto, C₁-C₆-alkylamino, hydroxy(C₁-C₆)alkylamino, amino(C₁-C₆)alkylamino)-6-(2,3-diamino-4-chlorobenzylamino)purine riboside,
and the pharmaceutically acceptable salts thereof with alkali metals, ammonium or amines, as well as their addition salts with acids.

The following substituted derivatives of the formula I are particularly preferred, namely:
2-((chloro, fluoro, C₁-C₆-alkylmercapto, C₁-C₆-alkylamino, hydroxy(C₁-C₆)alkylamino, amino(C₁-C₆)alkylamino)-6-(2-hydroxy-3-methoxybenzylamino)-purine riboside, 2-(chloro, fluoro, C₁-C₆-alkylmercapto, C₁-C₆-alkylamino, hydroxy(C₁-C₆)alkylamino, amino(C₁-C₆)alkylamino)-6-(2-hydroxy-5-methoxybenzylamino)purine riboside, 2-(chloro, fluoro, C₁-C₆-alkylmercapto, C₁-C₆-alkylamino, hydroxy(C₁-C₆)alkylamino, amino(C₁-C₆)alkylamino)-6-(2-hydroxy-3-methylbenzylamino)purine riboside, 2-(chloro, fluoro, C₁-C₆-alkylmercapto, C₁-C₆-alkylamino, hydroxy(C₁-C₆)alkylamino, amino(C₁-C₆)alkylamino)-6-(2-hydroxy-5-methylbenzylamino)purine riboside, 2-(chloro, fluoro, C₁-C₆-alkylmercapto, C₁-C₆-alkylamino, hydroxy(C₁-C₆)alkylamino, amino(C₁-C₆)alkylamino)-6-(2-hydroxy-3-chlorobenzylamino)purine riboside, 2-(chloro, fluoro, C₁-C₆-alkylmercapto, C₁-C₆-alkylamino, hydroxy(C₁-C₆)alkylamino, amino(C₁-C₆)alkylamino)-6-(2-hydroxy-5-chlorobenzylamino)purine riboside, 2-(chloro, fluoro, C₁-C₆-alkylmercapto, C₁-C₆-alkylamino, hydroxy(C₁-C₆)alkylamino, amino(C₁-C₆)alkylamino)-6-(2-hydroxy-5-fluorobenzyl-amino)purine riboside, 2-(chloro, fluoro, C₁-C₆-alkylmercapto, C₁-C₆-alkylamino, hydroxy(C₁-C₆)alkylamino, amino(C₁-C₆)alkylamino)-6-(2-amino-5-chlorobenzylamino)purine riboside, 2-(chloro, fluoro, C₁-C₆-alkylmercapto, C₁-C₆-alkylamino, hydroxy(C₁-C₆)alkylamino, amino(C₁-C₆)alkylamino)-6-(2-amino-5-fluorobenzylamino)purine riboside 2-(chloro, fluoro, C₁-C₆-alkylmercapto, C₁-C₆-alkylamino, hydroxy(C₁-C₆)alkylamino, amino(C₁-C₆)alkylamino)-6-(2-hydroxy-5-aminobenzylamino)purine riboside,
and the acceptable salts thereofthereof with alkali metals, ammonium or amines, as well as their addition salts with acids.

Another aspect of the invention are the 2-substituted-6-benzylaminopurine riboside derivatives of the general formula **I** for use as neutrophil survival factors based on inhibition of neutrophil apoptosis. Compounds according to this invention are particularly useful for therapeutic modulation of apoptosis and the inflammatory activity of neutrophils in terms of their suppression. Aspect of the invention are 2-substituted 6-(substituted benzylamino)purine ribosides of the general formula I for use as factors prolonging lifetime and activity of neutrophils, their mobility and ability to adhere vascular endothelium and migrate through it into the blood. The limitations of this process by the compounds according to this invention leads to the suppression of inflammation.

This invention further concerns the use of 2-substituted-6-(substituted benzylamino)purine riboside derivatives of formula **I** as anti-apoptotic factors of plant and animal cells in tissue cultures.

Substituted 6-benzylaminopurine ribosides of the general formula **I** have modulatory properties in the regulation of apoptosis and viability of neutrophils, due to which they may be used in the treatment or prophylaxis of diseases caused by reducing the viability of neutrophils, selected from a group including ANCA-associated vasculitis, glomerulonephritis-induced progressive renal failure (ESRF), neutropenia, emphysema, familial Mediterranean fever (FMF), arthralgia, peritonitis and amyloidosis. The compounds according to this invention are also useful as anti-inflammatory agents for the treatment and prophylaxis of chronic inflammatory diseases.

The compounds of the formula **I** are used in unmodified form or, preferably, together with the auxiliary substances conventionally employed in the art of formulation. To this end they are conveniently formulated in a known manner to emulsifiable concentrates, coatable pastes, tablets, capsules, directly sprayable or dilutable solutions, dilute emulsions, soluble powders, dusts, granulates, and also encapsulations, e.g. polymeric substances. The compositions may also contain further auxiliary substances such as stabilizers, antifoams, viscosity factors, binders or thickening agents, or other formulations for obtaining desired properties.

The compounds of the formula **I** can be mixed with other inflammatory-type drugs, resulting in some unexpected synergistic activities.

### COMPOSITIONS

Suitable routes for administration include oral, rectal, topical (including dermal, ocular, buccal and sublingual), vaginal and parenteral (including subcutaneous, intramuscular, intravitreous, intravenous, intradermal, intrathecal and epidural) way. The preferred route of administration will depend upon the condition of the patient, the toxicity of the compound and the type and site of infection, among other considerations known to the clinician.

The therapeutic compositions comprise about 1% to about 95% by weight of the active ingredient, single-dose forms of administration preferably comprising about 20% to about 90% by weight of the active ingredient and administration forms, which are not single-dose preferably comprising about 5% to about 20% of the active ingredient. Unit dose forms may be, for example, coated tablets, tablets, ampoules, vials, suppositories or capsules. Other forms of administration are, for example, ointments, creams, pastes, foams, tinctures, lipsticks, drops, sprays, dispersions and the like. Examples are capsules containing from about 0.05 g to about 1.0 g of the active ingredient.

The pharmaceutical compositions are prepared in a manner known per se, for example by means of conventional mixing, granulating, coating, dissolving or lyophilizing processes.

Preferably, solutions of the active ingredient, and in addition also suspensions or dispersions, especially isotonic aqueous solutions, dispersions or suspensions, are used, if being possible for these to be prepared before use, for example in the case of lyophilised compositions which comprise the active substance by itself or together with a carrier, for example mannitol. The pharmaceutical compositions can be sterilised and/or comprise excipients, for example preservatives, stabilisers, wetting agents and/or emulsifiers, solubilizing agents, salts for regulating the osmotic pressure and/or buffers, and they are prepared in a manner known per se, for example by means of conventional dissolving or lyophilising processes. The solutions or suspensions mentioned can comprise viscosity-increasing substances, such as sodium carboxymethylcellulose, dextran, polyvinylpyrrolidone or gelatine.

Suspensions in oil comprise, as the oily component, the vegetable, synthetic or semi-synthetic oils customary for injection purposes. Oils which may be mentioned are, in particular, liquid fatty acid esters which contain, as the acid component, a long-chain fatty acid having 8 - 22, in particular 12-22, carbon atoms, for example lauric acid, tridecylic acid, myristic acid, pentadecylic acid, palmitic acid, margaric acid, stearic acid, arachidonic acid, behenic acid or corresponding unsaturated acids, for example oleic acid, elaidic acid, euric acid, brasidic acid or linoleic acid, if appropriate with the addition of antioxidants, for example vitamin E, β-carotene or 3,5-di-*tert*-butyl-4-hydroxytoluene. The alcohol component of these fatty acid esters has not more than 6 carbon atoms and is mono- or polyhydric, for example mono-, di- or trihydric alcohol, for example methanol, ethanol, propanol, butanol, or pentanol, or isomers thereof, but in particular glycol and glycerol. Fatty acid esters are, for example: ethyl oleate, isopropyl myristate, isopropyl palmitate, "Labrafil M 2375" (polyoxyethylene glycerol trioleate from Gattefoseé, Paris), "Labrafil M 1944 CS" (unsaturated polyglycolated glycerides prepared by an alcoholysis of apricot kernel oil and made up of glycerides and polyethylene glycol esters; from Gattefoseé, Paris), "Labrasol" (saturated polyglycolated glycerides prepared by an alcoholysis of TCM and made up of glycerides and polyethylene glycol esters; from Gattefoseé, Paris) and/or "Miglyol 812" (triglyceride of saturated fatty acids of chain length C₈ to C₁₂ from Hüls AG, Germany), and in particular vegetable oils, such as cottonseed oil, almond oil, olive oil, castor oil, sesame oil, soybean oil and, in particular, groundnut oil.

The preparation of the injection compositions is carried out in the customary manner under sterile conditions, as are bottling, for example into ampoules or vials, and closing of the containers.

For example, pharmaceutical compositions for oral use can be obtained by combining the active ingredient with one or more solid carriers, if appropriate granulating the resulting mixture, and, if desired, processing the mixture or granules to tablets or coated tablet cores, if appropriate by addition of additional excipients. Suitable carriers are, in particular, fillers, such as sugars, for example lactose, sucrose, mannitol or sorbitol, cellulose preparations and/or calcium phosphates, for example tricalcium diphosphate, or calcium hydrogen phosphate, and furthermore binders, such as starches, for example maize, wheat, rice or potato starch, methylcellulose, hydroxypropylmethylcellulose, sodium carboxymethylcellulose and/or polyvinylpyrrolidine, and/or, if desired, desintegrators, such as the above mentioned starches, and furthermore carboxymethyl-starch, cross-linked polyvinylpyrrolidone, alginic acid or a salt thereof, such as sodium alginate. Additional excipients are, in particular, flow regulators and lubricants, for example salicylic acid, talc, stearic acid or salts thereof, such as magnesium stearate or calcium stearate, and/or polyethylene glycol, or derivatives thereof.

Coated tablet cores can be provided with suitable coatings which, if appropriate, are resistant to gastric juice, the coatings used being, inter alia, concentrated sugar solutions, which, if appropriate, comprise gum arabic, talc, polyvinylpyrrolidine, polyethylene glycol and/or titanium dioxide, coating solutions in suitable organic solvents or solvent mixtures or, for the preparation of coatings which are resistant to gastric juice, solutions of suitable cellulose preparations, such as acetylcellulose phthalate or hydroxypropylmethylcellulose phthalate. Dyes or pigments can be admixed to the tablets or coated tablet coatings, for example for identification or characterisation of different doses of active ingredient.

Pharmaceutical compositions, which can be used orally, are also hard capsules of gelatine and soft, closed capsules of gelatine and a plasticiser, such as glycerol or sorbitol. The hard capsules can contain the active ingredient in the form of granules, mixed for example with fillers, such as maize starch, binders and/or lubricants, such as talc or magnesium stearate, and stabilisers if appropriate. In soft capsules, the active ingredient is preferably dissolved or suspended in suitable liquid excipients, such as greasy oils, paraffin oil or liquid polyethylene glycol or fatty acid esters of ethylene glycol or propylene glycol, it being likewise possible to add stabilisers and detergents, for example of the polyethylene sorbitan fatty acid ester type.

Other oral forms of administration are, for example, syrups prepared in the customary manner, which comprise the active ingredient, for example, in suspended form and in a concentration of about 5% to 20% by weight, preferably about 10% by weight or in a similar concentration which results in a suitable individual dose, for example, when 5 or 10 mL are measured out. Other forms are, for example, also pulverulent or liquid concentrates for preparing of shakes, for example in milk. Such concentrates can also be packed in unit dose quantities.

Pharmaceutical compositions, which can be used rectally, are, for example, suppositories that comprise a combination of the active ingredient with a suppository base. Suitable suppository bases are, for example, naturally occurring or synthetic triglycerides, paraffin hydrocarbons, polyethylene glycols or higher alkanols. Compositions which are suitable for parental administration are aqueous solutions of an active ingredient in water-soluble form, for example of water-soluble salt, or aqueous injection suspensions, which comprise viscosity-increasing substances, for example sodium carboxymethylcellulose, sorbitol and/or dextran, and, if appropriate, stabilizers. The active ingredient can also be present here in the form of a lyophilisate, if appropriate, together with excipients, and be dissolved before parenteral administration by addition of suitable solvents. Solutions such as are used, for example, for parental administration can also be used as infusion solutions. Preferred preservatives are, for example, antioxidants, such as ascorbic acid, or microbicides, such as sorbic or benzoic acid.

Ointments are oil-in-water emulsions which comprise not more than 70%, preferably 20 - 50% by weight of water or aqueous phase. The fatty phase consists, in particular, hydrocarbons, for example vaseline, paraffin oil or hard paraffins, which preferably comprise suitable hydroxy compounds, such as fatty alcohols or esters thereof, for example cetyl alcohol, or wool wax alcohols, such as wool wax, to improve the water-binding capacity. Emulsifiers are corresponding lipophilic substances, such as sorbitan fatty acid esters (Spans), for example sorbitan oleate and/or sorbitan isostearate. Additives to the aqueous phase are, for example, humectants, such as polyalcohols, for example glycerol, propylene glycol, sorbitol and/or polyethylene glycol, or preservatives and odoriferous substances.

Fatty ointments are anhydrous and comprise, as the base, in particular, hydrocarbons, for example paraffin, vaseline or paraffin oil, and furthermore naturally occurring or semi-synthetic fats, for example hydrogenated coconut-fatty acid triglycerides, or, preferably, hydrogenated oils, for example hydrogenated groundnut or castor oil, and furthermore fatty acid partial esters of glycerol, for example glycerol mono- and/or distearate, and for example, the fatty alcohols. They also contain emulsifiers and/or additives mentioned in connection with the ointments which increase uptake of water.

Creams are oil-in-water emulsions, which comprise more than 50% of water. Oily bases used are, in particular, fatty alcohols, for example lauryl, cetyl or stearyl alcohols, fatty acids, for example palmitic or stearic acid, liquid to solid waxes, for example isopropyl myristate, wool wax or beeswax, and/or hydrocarbons, for example vaseline (petrolatum) or paraffin oil. Emulsifiers are surface-active substances with predominantly hydrophilic properties, such as corresponding nonionic emulsifiers, for example fatty acid esters of polyalcohols or ethyleneoxy adducts thereof, such as polyglyceric acid fatty acid esters or polyethylene sorbitan fatty esters (Tweens), and furthermore polyoxyethylene fatty alcohol ethers or polyoxyethylene fatty acid esters, or corresponding ionic emulsifiers, such as alkali metal salts of fatty alcohol sulphates, for example sodium lauryl sulphate, sodium cetyl sulphate or sodium stearyl sulphate, which are usually used in the presence of fatty alcohols, for example cetyl stearyl alcohol or stearyl alcohol. Additives to the aqueous phase are, inter alia, agents which prevent the creams from drying out, for example polyalcohols, such as glycerol, sorbitol, propylene glycol and/or polyethylene glycols, and furthermore preservatives and odoriferous substances. Pastes are creams and ointments having secretion-absorbing powder constituents, such as metal oxides, for example titanium oxide or zinc oxide, and furthermore talc and/or aluminium silicates, which have the task of binding the moisture or secretions present.

Foams are administered from pressurised containers and they are liquid oil-in-water emulsions present in aerosol foam. As the propellant gases, halogenated hydrocarbons, such as polyhalogenated alkanes, for example dichlorofluoromethane and dichlorotetrafluoroethane, or, preferably, non-halogenated gaseous hydrocarbons air, N₂O or carbon dioxide are used. The oily phases used are, inter alia, those mentioned above for ointments and creams, and the additives mentioned there are likewise used.

Tinctures and solutions usually comprise an aqueous-ethanolic base to which, humectants for reducing evaporation, such as polyalcohols, for example glycerol, glycols and/or polyethylene glycol, and re-oiling substances, such as fatty acid esters with lower polyethylene glycols, i.e. lipophilic substances soluble in the aqueous mixture to substitute the fatty substances removed from the skin with ethanol, and, if necessary, other excipients and additives, are admixed.

Further provided are veterinary compositions comprising at least one active ingredient as above defined together with a veterinary carrier therefor. Veterinary carriers are materials for administering the composition and may be solid, liquid or gaseous materials, which are inert or acceptable in the veterinary art and are compatible with the active ingredient. These veterinary compositions may be administered orally, parenterally or by any other desired route.

Disclosed is also a process or method for treatment of the disease states mentioned above. The compounds can be administered prophylactically or therapeutically as such or in the form of pharmaceutical compositions, preferably in an amount, which is effective against the diseases mentioned. With a warm-blooded animal, for example a human, requiring such treatment, the compounds are used, in particular, in the form of pharmaceutical composition. A daily dose of about 0.1 to about 5 g, preferably 0.5 g to about 2 g, of a compound of the present invention is administered here for a body weight of about 70 kg.

### Brief Description of Drawings

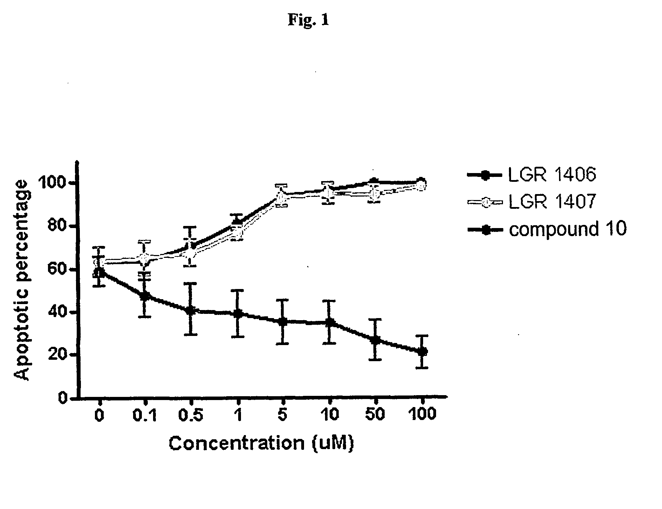

Fig. 1 displays inhibition of neutrophil apoposis by compound **10.** Neutrophils were cultured at 2 x 10⁶ cells/ml for 20h in RPMI1640 medium containing human serum and antibiotics and a range of concentrations of compound **10.** Apoptosis was determined by differential Giemsa staining and light microscopy to determine cells with an apoptotic nuclear morphology. Data are mean ± SD of 5 separate experiments.

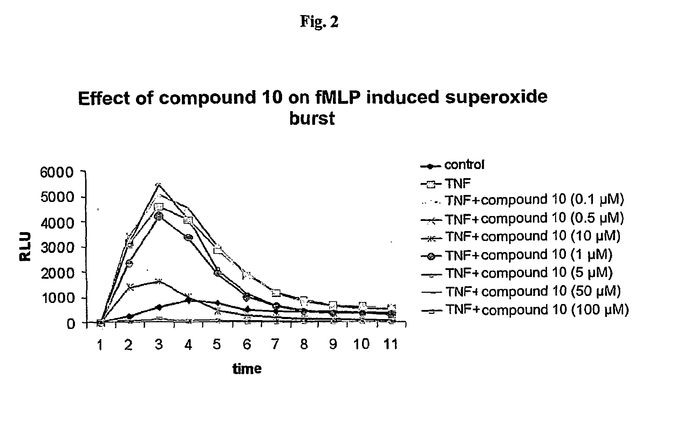

Fig. 2 shows inhibition of TNF primed neutrophil superoxide generation by compound **10.** Neutrophils were cultured at 2 x 10⁶ cells/ml in RPMI1640 medium containing human serum antibiotics and 20 ng/ml TNFα for 10 minutes prior to addition of a range of concentrations of compound **10.** Generation of superoxide was determined over a 10 minute period using a lucigenin based assay. The area under the curve was calculated to give superoxide generation over the whole assay period. The image shows a representative plot of fluorescence increase, representative of 5 separate experiments.

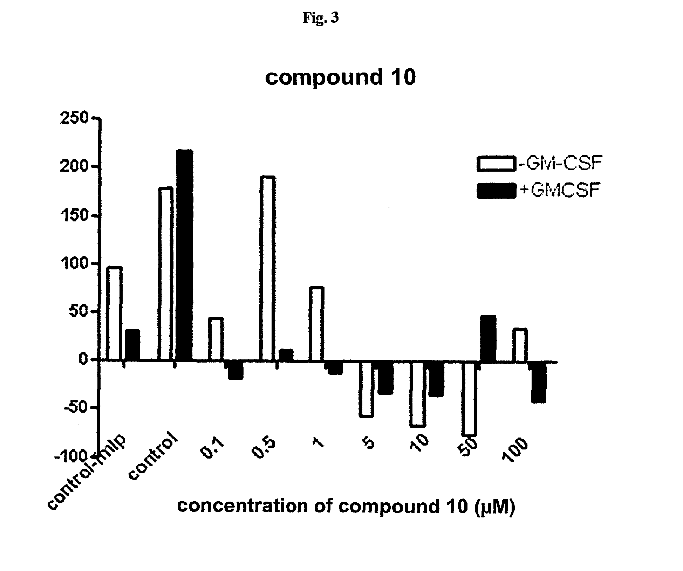

Fig. 3 shows inhibition of IL-8 release from TNF-primed neutrophils by compound **10.** Neutrophils were cultured at 2 x 10⁶ cells/ml in RPMI1640 medium containing human serum antibiotics and 20 ng/ml TNFα for 10 minutes prior to addition of a range of concentrations of compound **10.** Release of IL8 was monitored after a 4h period by ELISA. Data are mean of duplicate measurements representative of 5 separate experiments.

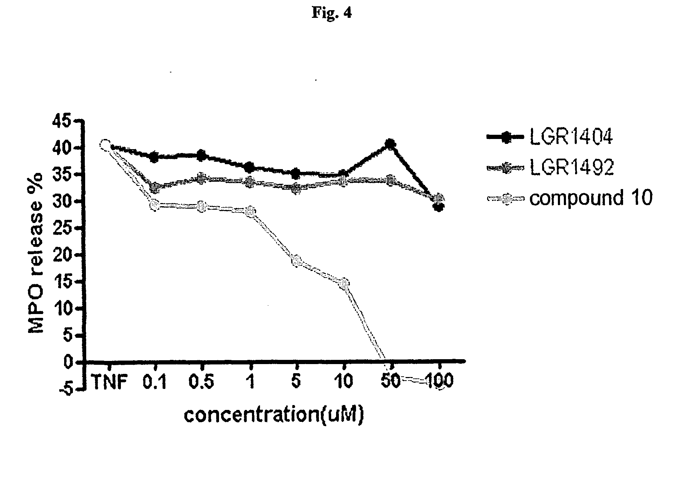

Fig. 4 shows inhibition of TNF-primed neutrophil degranulation by compound **10.** Neutrophils were cultured at 2 x 10⁶ cells/ml in RPMI1640 medium containing human serum antibiotics and 20 ng/ml TNFα for 10 minutes prior to addition of a range of concentrations of compound **10.** Release of MPO was monitored after a 30 minute period by ELISA. Data are mean of duplicate measurements representative of 5 separate experiments.

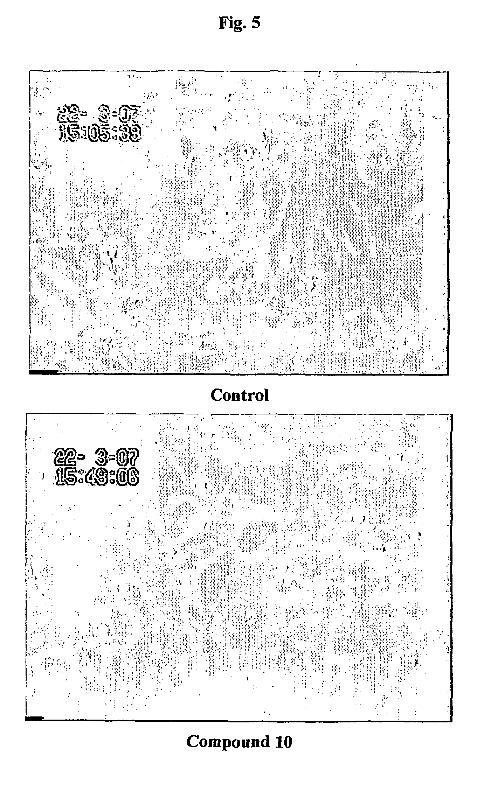

Fig. 5 displays positive effects by compound **10** on neutrophil adhesion and migration under flow.

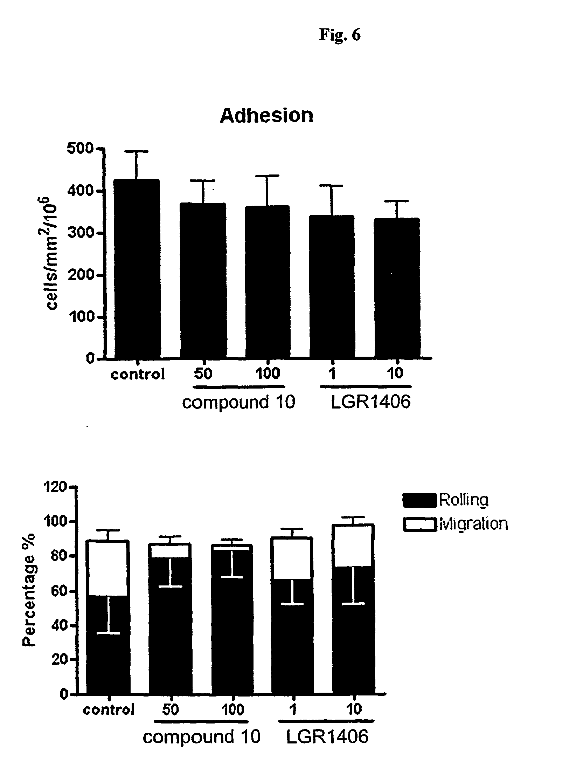

Fig. 6 shows positive effects of compound **10** on inhibition of neutrophil adhesion and migration under flow. Neutrophils were introduced into a flow based cell adhesion instrument at 5 x 10⁶ cells/ml in medium contaning 50 or 100 µM of compound **10.** The capillary system is lined with TNF-primed HUVEC and cell adhesion, rolling and transmigration is monitored by time-lapse video microscopy. Data are expressed as cells adhered to HUVEC per mm², per 10⁶ cells (left panel) or % of cells rolling or migrated (right panel). LGR 1406 (N-5-(2-aminocyclohexyl)-N-7-benzyl-3-isopropyl-1(2)H-pyrazolo[4,3-d]pyrimidine-5,7-diamine, Sroka et al. Mol Pharmacol. 2010; 77(2):255-61) was used as a negative control. Data are mean ± SD of 3 separate experiments.

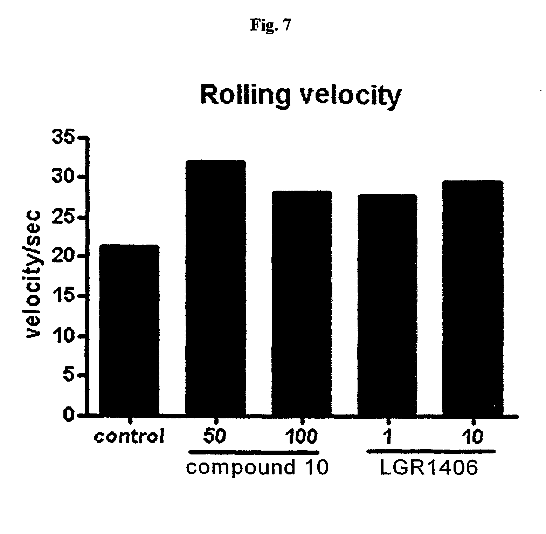

Fig. 7 shows positive effects of compound **10** on neutrophil rolling velocity. LGR 1406 as a negative control.

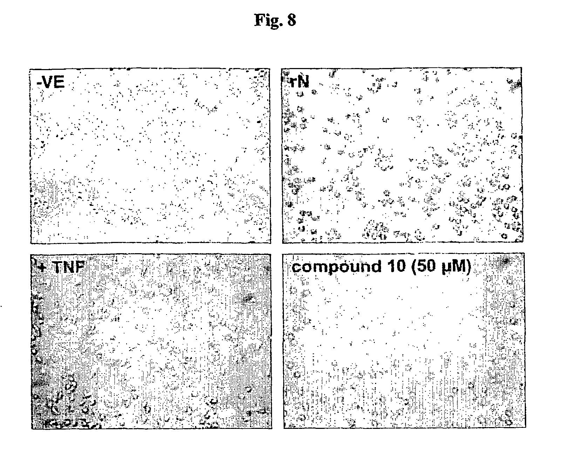

Fig. 8 shows that compound **10** reduces neutrophil spreading in Fibronectin.

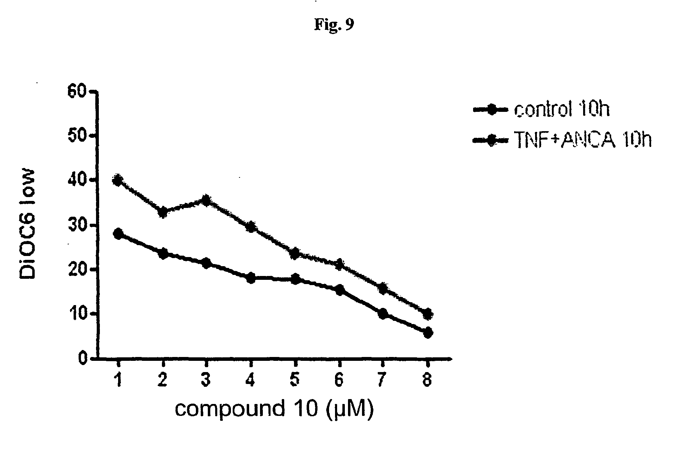

Fig. 9 shows inhibition of ANCA induced neutrophil apoptosis by compound **10.** Neutrophils were cultured at 2 x 10⁶ cells/ml for 10h in RPMI1640 medium containing human serum and antibiotics and a range of concentrations of compound **10** in the absence or presence of ANCA. Apoptosis was determined by flow cytometry after DiOC6 staining.

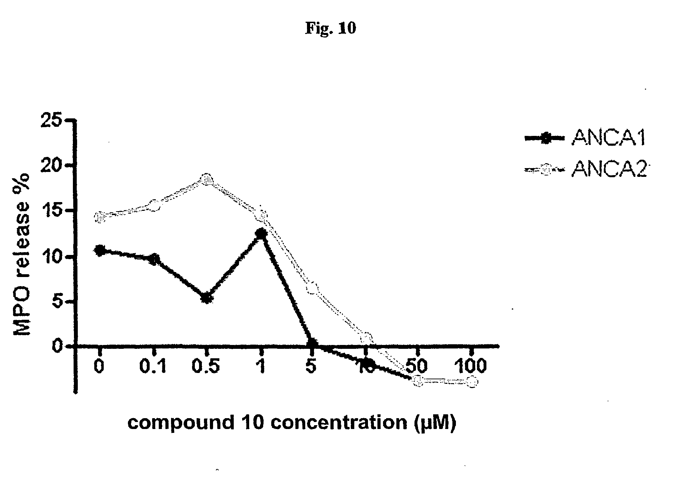

Fig. 10 Inhibition of ANCA induced neutrophil degranulation by compound **10.** Neutrophils were cultured at 2 x 10⁶ cells/ml in RPMI1640 medium containing human serum antibiotics and 20 ng/ml TNFα for 10 minutes prior to addition of ANCA (1 = MPO ANCA; 2 = PR3 ANCA) and a range of concentrations of compound **10.** Release of MPO was monitored after a 30 minute period by ELISA.

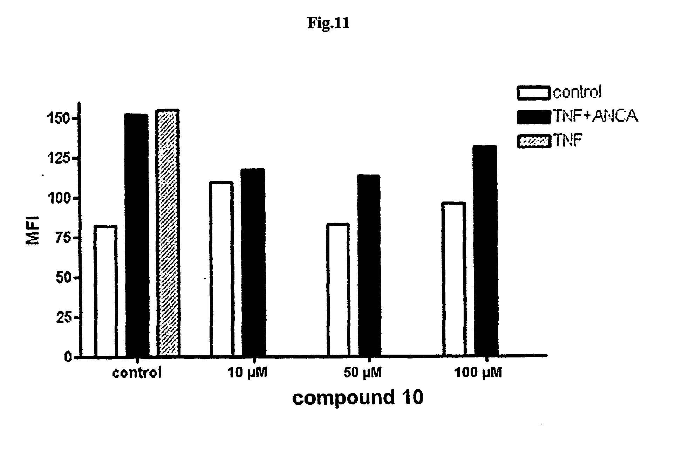

Fig. 11 Inhibition of ANCA induced neutrophil ROS production by compound **10.** Neutrophils were cultured at 2 x 10⁶ cells/ml in RPMI1640 medium containing human serum antibiotics and 20 ng/ml TNFα for 10 minutes prior to addition of ANCA and a range of concentrations of compound **10.** Generation of superoxide was determined over a 10 minute period using a lucigenin based assay. The area under the curve was calculated to give superoxide generation over the whole assay period.

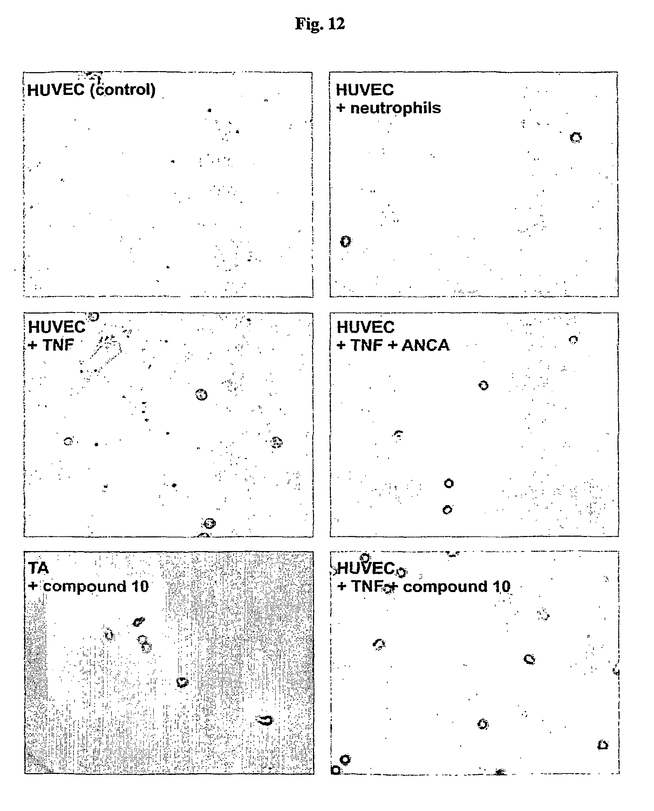

Fig. 12 displays principle of compound **10** protection from ANCA induced damage of endothelial cells (HUVEC). HUVEC were cultured to confluence in tissue culture plates and incubated overnight with neutrophils treated with 20 ng/ml TNFα (+TNF), TNF and 50 µM compound **10** (TNF+LGR), TNF and ANCA (TA) or TNF and ANCA and 50 µM compound **10** (TA+LGR). Viable cells remain attached to the plastic wells and are visualised by phase contrast light microscopy. The image shown is representative of 3 separate experiments.

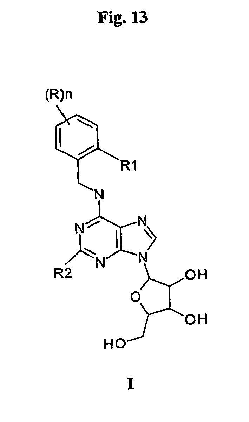

Fig. 13 shows the general formula I.

### Examples of Carrying out the Invention

The invention is further illustrated by the following examples, which should not be construed as further limiting.

The starting material for the compounds of the formula **I** is 2,6-dichloropurine riboside. Another starting material can be 2-amino-6-chloropurine riboside or 2-fluoro-6-chloropurine riboside, synthesised from 2-amino-6-chloropurine riboside by reaction with tetrafluoroboric acid in the presence of sodium nitrite aqueous solution (Beach et al., 1992). Yet another starting material can be 2-methyl-6-chloropurine riboside, which can be prepared from 6-chloro-2-iodopurine riboside by Stille's cross coupling (Kim et al., 2003).

Starting substituted benzyl or phenylamines, not commercially available (others obtained *via* Sigma Aldrich or Fluorochem), were prepared from the corresponding aldehydes in the presence of a suitable metal catalyst. These, which have one or more hydroxyl groups, may also be prepared by demethylation of appropriate methoxyderivatives using 48% HBr in N₂ atmosphere.

Elemental analyses (C, H and N) were performed on an EA1108 CHN analyser (Thermo Finnigan). The melting points were determined on a BÜCHI Melting Point B-540 apparatus and are uncorrected. Analytical thin layer chromatography (TLC) was carried out using silica gel 60 WF₂₅₄ plates (Merck), solvent CHCl₃:MeOH:conc. NH₄OH (8:2:0.2, v/v/v). ES+ mass spectra were recorded using direct probe on Waters ZMD 2000 using direct probe. The mass monitoring interval was 10-1500 amu. The spectra were collected using 3.0 second cyclical scans and applying sample cone voltage 25 V at source block temperature 150 °C, desolvation temperature 80 °C and desolvation gas flow rate 200 l/hour. The mass spectrometer was directly coupled to a MassLynx data system.EI mass spectra were measured on Polaris Q (Finnigan) mass spectrometer using direct inlet (DIP) The mass monitoring interval was 50 - 1000 amu using cyclic scans (3.0 s), at 70 eV, temperature gradient 40-450 °C. NMR spectra were measured in a Bruker Avance AV 300 spectrometer operating at a temperature of 300 K and a frequency of 300.13 MHz (¹H) and 75.48 MHz (¹³C), respectively. Samples were prepared by dissolving the compounds in DMSO-d₆. Tetramethylsilane (TMS) was used as the internal standard. The samples were prepared by the dissolution of the substances in DMSO-*d₆*.

### EXAMPLE 1

### 2-Chloro-6-(2,4-dimethylbenzylamino)purine riboside

2,4-dimethylbenzylamine (8.35 g; 0.05 mol) was added to a suspension of 2,6-dichloropurine riboside (16.0 g; 0.05 mol) in *n*-propanol (100 mL) and *N,N'* ethyldiisopropylamine (6.44 g; 0.05 mol) was added. The reaction mixture was stirred at 100 °C for 4 hours. After cooling to room temperature the precipitate was filtered off, washed with cold n-propanol (2x10 mL) and water (3x10 mL) and dried in the drying oven at 60°C into constant weight. Yield: 6.26 g of yellowish substance (84.9%). TLC (chloroform-methanol; 85:15): one single spot; free of starting material, HPLC purity: 98+%

### EXAMPLE 2

### 2-Chloro-6-(2,3-dihydroxybenzylamino)purine riboside

2,3-dihydroxybenzylamine (4.17 g; 0.03 mol) was added to a suspension of 2,6-dichloropurine riboside (6.42 g; 0.02 mol) in n-butanol (40 mL) and triethylamine (5.7 g; 0.05 mol) was added. The reaction mixture was stirred at 110 °C for 3 hours. After cooling to room temperature was reaction mixture stirred at 0 °C for two hours, the precipitate was filtered off, washed with cold n-butanol (2x10mL) and water (3x10mL) and dried in the drying oven at 60°C into constant weight. Yield: 3.99 g of yellowish substance (84.9 %). Crude product was crystallized from isopropanol and the yield of pure substance was 2.85 g. TLC (chloroform-methanol; 85:15): one single spot; free of starting material, HPLC purity: 98+%

### EXAMPLE 3

### 2-Chloro-6-(2,3-dimethoxybenzylamino)purine riboside

2,3-dimethylbenzylamine (8.35 g; 0.05 mol) was added to a suspension of 2,6-dichloropurine riboside (16 g; 0.05 mol) in n-propanol (100 mL) and *N,N'* ethyldiisopropylamine (6.44 g; 0.05 mol) was added. The reaction mixture was stirred at 100 °C for 4 hours. Reaction mixture was evaporated and mixed with ethyl acetate and 0.5 M HCl. Ethyl acetate phase was washed with water, dried over MgSO₄ and evaporated to yellow solid (3.92 g). Crude product was purified by flash chromatography. Yield: 2.70 g of yellowish substance. TLC (chloroform-methanol; 85:15): one single spot; free of starting material, HPLC purity: 98+%

### EXAMPLE 4

### 2-Chloro-6-(2,5-dihydroxybenzylamino)purine riboside

The substance was prepared by the reaction of 2,6-dichloropurine riboside (6.42 g, 0.02 mol) with 2,5-dihydroxybenzylamine (4.17 g, 0.03 mol) in the presence of N,N'- ethyldiisopropylamine (6.44 g, 0.05 mol) in *n*-butanol (40 mL) for 4 hrs at 90 °C. The reaction mixture was cooled to room temperature, crystaline solid was filtrated off, washed with cold *n*-butanol (3x10 mL), water (3x10 mL) and dried in drying oven into constant weight. TLC (chloroform:methanol, 85:15): one single spot, HPLC purity: 98+%

### EXAMPLE 5

### 2-Chloro-6-(2-hydroxy-3-methoxybenzylamino)purine riboside

2,6-dichloropurine riboside (6.42 g, 0.02 mol) was mixed with 2-hydroxy-3-methoxybenzylamine (4.17 g, 0.03 mol) and with triethylamine (7 mL, 0.05 mol) in *n*-pentanol (40 mL). Reaction mixture was stirred at 100 °C for 4 hrs and then was cooled to room temperature. White product was filtered off, washed with cold isopropanol (2x10 mL) and water (3x10 mL). Crude product was purified by crystallization from methanol in the presence of charcoal and white crystals were obtained. Yield: 4.36g, TLC (chloroform:methanol, 85:15): one single spot, no starting substance, HPLC purity: 98+%.

### EXAMPLE 6

### 2-chloro-6-(2-hydroxybenzylamino)purine riboside

Triethylamine (4 mL, 0.035 mol) was added to a suspension of 2,6-dichloropurine riboside (2 g, 0.006 mol) and 2-hydroxybenzylamine (0.76 g, 0.005 mol) in *n-*propanol (40 mL). The reaction mixture was stirred at 100 °C for 4 hrs and then allowed to cool to RT. White precipitate was collected, washed with isopropanol (2x10 mL), water (3x10 mL). The crude product was purified by crystallization from isopropanol in the presence of activated charcoal to give almost white crystals. Yield: 65%. TLC (chloroform:methanol, 85:15): one single spot, free of starting material, HPLC purity: 98+%, ¹H NMR (300 MHz, DMSO-*d*6) δ ppm 3.58 (br. S., 1H); 3.65 (br. s., 1H); 3.95 (br. s., 1H); 4.13 (br. s., 1H); 4.41 - 4.70 (m, 2H); 5.05 (br. s., 1H); 5.20 (d, *J*=4.57 Hz, 1H); 5.48 (d, *J*=6.04 Hz, 1H); 5.84 (d, *J*=5.49 Hz, 1H); 6.64 - 6.92 (m, 2H); 7.07 (d, *J*=6.95 Hz, 2H); 8.42 (br. s., 1H); 9.59 (s, 1H).

### EXAMPLE 7

### 2-Chloro-6-(2-methoxybenzylamino)purine riboside

To a suspension of 2,6-dichloropurine riboside (2 g, 0.006 mol) and 2-methoxybenzylamine (0.84 g; 0.005 mol) in n-pentanol (40 mL), triethylamine (7 mL; 0.05 mol) was added. The reaction mixture was stirred at 100°C for 4 hours and then was allowed to cool to room temperature. White precipitate was collected, washed with cold isopropanol (2x10 mL), water (3x10 mL). The crude product was purrified by crystallization from methanol in the presence of activated charcoal to give almost white crystals. Yield: 4.36g (77.8%). TLC (chloroform-methanol; 85:15): one single spot, free of starting material HPLC purity: 98+%, ¹H NMR (300 MHz, DMSO-*d*₆) δ ppm 3.50 - 3.79 (m, 6H); 3.95 (d, *J*=3.29 Hz, 1H); 4.04 - 4.30 (m, 2H); 4.46 - 4.81 (m, 3H); 4.86 - 5.15 (m, 1H); 5.20 (d, *J*=4.94 Hz, 1H); 5.48 (d, *J*=6.04 Hz, 1H); 5.83 (d, *J*=5.85 Hz, 1H); 6.68 - 6.85 (m, 1H); 6.85 - 7.00 (m, 2H); 7.05 - 7.39 (m, 1H); 8.42 (s, 1H);

### EXAMPLE 8

### 2-Chloro-6-(2-methoxy-4-hydroxybenzylamino)purine riboside

Triethylamine (4 mL, 0.035 mol) was added to a suspension of 2,6-dichloropurine riboside (2 g, 0.006 mol) and (2-methoxy-4-hydroxy)benzylamine hydrochloride (1.135 g, 0,006 mol) in *n*-propanol (40 mL). The reaction mixture was stirred at 100 °C for 4 hrs and then allowed to cool to room temperature. White precipitate was collected, washed with isopropanol (2x10 mL), water (3x10 mL). The crude product was purified by crystallization from isopropanol in the presence of activated charcoal to give almost white crystals. Yield: 65%. TLC (chloroform:methanol, 85:15): one single spot, free of starting material, HPLC purity: 98+%, ¹H NMR (300 MHz, DMSO-*d*₆) δ ppm 2.23 (br. s, 1H); 3.38 - 3.73 (m, 5H); 3.90 (d, *J*=3.29 Hz, 1H); 3.98 - 4.26 (m, 1H); 4.34 - 4.62 (m, 2H); 5.00 (t, *J*=5.49 Hz, 1H); 5.14 (d, *J*=4.94 Hz, 1H); 5.42 (d, *J*=6.04 Hz, 1H); 5.78 (d, *J*=5.49 Hz, 1H); 6.63 - 6.95 (m, 3H); 8.35 (s, 1H); 8.82 (s, 1H).

### EXAMPLE 9

### 2-Chloro-6-(2-hydroxy-5-chlorobenzylamino)purine riboside

Triethylamine (4 mL, 0.035 mol) was added to a suspension of 2,6-dichloropurine riboside (2 g, 0.006 mol) and (2-hydroxy-5-chloro)benzylamine hydrochloride (1.10 g, 0,005 mol) in *n*-propanol (40 mL). The reaction mixture was stirred at 100 °C for 4 hrs and then allowed to cool to RT. White precipitate was collected, washed with isopropanol (2x10 mL), water (3x10 mL). The crude product was purified by crystallization from isopropanol in the presence of activated charcoal to give almost white crystals. Yield: 65%. TLC (chloroform:methanol, 85:15): one single spot, free of starting material, HPLC purity: 98+%, ¹H NMR (300 MHz, DMSO-*d*₆) δ ppm 1.09 (t, *J*=7.04 Hz, 1H); 3.38 (d, *J*=6.95 Hz, 1H); 3.57 (br. s., 1H); 3.67 (d, *J*=12.26 Hz, 1H); 3.94 (br. s., 1H) 4.13 (br. s., 1 H); 4.45 - 4.65 (m, 1H); 5.03 (t, *J*=5.67 Hz, 1H); 5.18 (d, *J*=4.76 Hz, 1H); 5.47 (d, *J*=6.04 Hz, 1H); 5.84 (d, *J*=5.85 Hz, 1H); 6.83 (d, *J*=8.23 Hz, 1H); 7.00 - 7.18 (m, 1H); 8.44 (s, 1H); 8.74 (d, *J*=12.99 Hz, 1H); 9.92 (s, 1H).

### EXAMPLE 10

### 2-Chloro-6-(2-chlorobenzylamino)purine riboside

2,6-Dichloropurine riboside (6.42 g; 0.02 mol) was reacted with 2-chlorobenzylamine (4.26 g; 0.03 mol) in *n*-butanol (40 mL) in the presence of triethylamine (7 mL; 0.05 mol) at 100°C for 4 hours. After cooling to room temperature the reaction mixture was stirred at 0°C for 2 hours. The yellow precipitate was filtered off, washed with cold n-butanol (2x10mL), water (3x10mL) and dried in the drying oven at 60°C into constant weight. Yield: 3.69 g yellow crystalline powder (65.8%). The crude product was crystallized from isopropanol to give 2.85 g of pure substance. TLC (chloroform-methanol; 85:15): one single spot; free of starting material. HPLC purity: 98+%

### EXAMPLE 11

### 2-Chloro-6-(2-fluorobenzylamino)purine riboside

This substance was prepared in similar manner as example 11 by the reaction of 2,6-dichloropurine riboside with 2-fluorobenzylamine. The reaction mixture was then evaporated on rotary vacuum evaporator and the residue was partitioned between ethyl acetate and water. The organic layer was dried with MgSO₄, and evaporated to give a yellow solid (3.72 g). The crude product was purrified by flash chromatography. Yield: 2.20 g yellow crystalline powder. TLC (chloroform-methanol; 85:15): homogenous. HPLC purity: 98+%

**Table 1: Compounds Prepared by the Method of Examples 1-11**

| SUBSTITUENT | | | CHN ANALYSES | MS ANALYSES-ZMD | |
|---|---|---|---|---|---|
| | R6 | R2 | calculated/found [%] | [M-H]⁻ a) | [M+H]⁺b) |
| **1** | 2,3-dimethoxybenzylamino | chloro | C=50,5/50,4; H=4,9/4,9; N=15,5/15,7 | 450 | 452 |
| **2** | 2,4-dimethoxybenzylamino | chloro | C=50,5/50,4; H=4,9/4,9; N=15,5/15,7 | 450 | 452 |
| **3** | 2,6-dimethoxybenzylamino | chloro | C=50,5/50,3; H=4,9/4,9; | 450 | 452 |
| | | | N=15,5/15,8 | | |
| **4** | 2,4,5-trimethoxybenzylamino | chloro | C=49,9/50,3; H=5,0/4,9; N=14,5/14,9 | 480 | 482 |
| **5** | 2,6-dimethoxy-4-hydroxybenzylamino | chloro | C=48,8/48,3; H=4,7/4,7; N=14,5/14,9 | 466 | 468 |
| **6** | 2,3-dihydroxybenzylamino | chloro | C=48,2/48,0; H=4,3/4,4; N=16,5/16,7 | 422 | 424 |
| **7** | 2,5-dihydroxybenzylamino | chloro | C=48,8/47,9; H=4,3/4,2; N=16,5/16,9 | 422 | 424 |
| **8** | 2-chloro-5-methoxybenzylamino | chloro | C=47,4/47,1; H=4,2/4,2; N=15,4/15,9 | 454 | 456 |
| **9** | 2-methoxy-3-chlorobenzylamino | chloro | C=47,4/47,0; H=4,2/4,0; N=15,4/14,9 | 454 | 456 |
| **10** | 2-hydroxy-3-methoxybenzylamino | chloro | C=49,4/49,2; H=4,6/4,8; N=16,0/15,8 | 436 | 438 |
| **11** | 2-hydroxy-4-methoxybenzylamino | chloro | C=49,4/49,0; H=4,6/4,8; N=16,0/15,6 | 436 | 438 |
| **12** | 2-hydroxy-5-methoxybenzylamino | chloro | C=49,4/49,1; H=4,6/4,7; N=16,0/15,6 | 436 | 438 |
| **13** | 2-hydroxy-3-methylbenzylamino | chloro | C=51,3/51,0; H=4,8/4,7; N=16,6/16,5 | 420 | 422 |
| **14** | 2-hydroxy-5-methylbenzylamino | chloro | C=51,3/51,1; H=4,8/4,9; N=16,6/16,1 | 420 | 422 |
| **15** | 2-hydroxy-3-chlorobenzylamino | chloro | C=46,2/46,1; H=3,9/4,1; N=15,8/16,0 | 420 | 422 |
| **16** | 2-methoxy-3-hydroxybenzylamino | chloro | C=49,4/49,3; H=4,6/4,6; N=16,0/15,7 | 436 | 438 |
| **17** | 2,4-dimethylbenzylamino | chloro | C=54,4/54,1; H=5,3/5,4; N=16,7/16,5 | 418 | 420 |
| **18** | 2-hydroxy-5-aminobenzylamino | chloro | C=49,5/49,3; H=4,9/5,0; N=19,2/19,0 | 435 | 437 |
| **19** | 2-amino-5-methoxybenzylamino | chloro | C=49,5/49,6; H=4,9/4,7; N=19,2/18,8 | 435 | 437 |
| **20** | 2-amino-3-methylbenzylamino | chloro | C=51,4/51,6; H=5,0/4,9; N=20,0/20,2 | 419 | 421 |
| **21** | 2-methoxy-3-hydroxybenzylamino | chloro | C=49,4/49,0; H=4,6/4,8; N=16,0/15,6 | 436 | 438 |
| **22** | 2-hydroxybenzylamino | chloro | C=50,1/50,3; H=4,5/4,8; N=17.2/17,7 | 407 | 409 |
| **23** | 2-aminobenzylamino | chloro | C=53.1/53.2; H=5.7/5.6; N=20.7/20.9 | 406 | 408 |
| **24** | 2-methoxybenzylamino | chloro | C=51,3/51,2; H=4,8/4,9; N=16.6/16,5 | 421 | 423 |
| **25** | 2-chlorobenzylamino | chloro | C=47,9/48,2; H=4,0/4,1; N=16.4/16,5 | 425 | 427 |
| **26** | 2-fluorobenzylamino | chloro | C=49,8/49,5; H=4,2/4,1; N=17.1/17.5 | 409 | 411 |
| **27** | 2-methylbenzylamino | chloro | C=56.2/56.3; H=5.9/6.0; | 405 | 407 |
| | | | N=17.2/17,5 | | |
| **28** | 2-methoxy-4-hydroxybenzylamino | chloro | C=49,4/49,2; H=4,6/4,5; N=16,0/15,5 | 436 | 438 |
| **29** | 2-hydroxy-5-chlorobenzylamino | chloro | C=46,2/46,3; H=3,9/4,0; N=15,8/15,9 | 420 | 422 |
| **30** | 2-hydroxy-5-fluorobenzylamino | chloro | C=50.8/51.0; H=5.0/5.1; N=16.4/16.3 | 424 | 426 |
| **31** | 2-hydroxy-5-aminobenzylamino | chloro | C=51.1/51.0; H=5.5/5.3; N=19.9/19.7 | 422 | 424 |
| **32** | 2,4-difluorobenzylamino | chloro | C=50.5/50.3; H=4.7/4.9; N=16.4/16.6 | 427 | 429 |
| **33** | 2,3,6-trifluorobenzylamino | chloro | C=48.5/48.4; H=4.3/4.5; N=15.7/15.9 | 445 | 447 |

### EXAMPLE 12

### 2-Fluoro-6-(2-methoxybenzylamino)purine riboside

This compound was prepared by the reaction of 2-fluoro-6-chloropurine riboside (1.7g; 0.01mol), 2-methoxybenzylamine (1.23g; 0.01mol and triethylamine (3.5mL; 0.025mol) in *n*-butanol (20mL) at 90°C for 4 hours. The reaction mixture was then cooled to room temperature. White precipitate was filtered off, rinsed with *n*-butanol (3x10mL) and water (3x10mL) and dried in the drying oven into constant weight. Yield: 5.55g (61%) of yellowish crystalline powder. TLC (chloroform-methanol; 85:15): one single spot. HPLC purity: 99+%.

**Table 2: Compounds Prepared by the Method of Example 12**

| SUBSTITUENT | | | CHN ANALYSES | MS ANALYSES-ZMD | |
|---|---|---|---|---|---|
| | R6 | R2 | calculated/found [%] | [M-H]⁻ a) | [M+H]⁺b) |
| **34** | 2,3-dimethoxybenzylamino | fluoro | C=52,4/52,3; H=5,1/5,1; N=16,1/15,9 | 434 | 436 |
| **35** | 2,4-dimethoxybenzylamino | fluoro | C=52,4/52,3; H=5,1/5,0; N=16,1/16,0 | 434 | 436 |
| **36** | 2,4,5-trimethoxybenzylamino | fluoro | C=51,6/51,4; H=5,2/5,2; N=15,1/14,7 | 464 | 466 |
| **37** | 2,6-dimethoxy-4-hydroxybenzylamino | fluoro | C=50,6/50,1; H=4,9/4,9; N=15,5/15,8 | 450 | 452 |
| **38** | 2,3-dihydroxybenzylamino | fluoro | C=50,1/49,6; H=4,5/4,5; N=17,2/16,8 | 406 | 408 |
| **39** | 2,5-dihydroxybenzylamino | fluoro | C=50,1/49,8; H=4,5/4,5; N=17,2/16,9 | 406 | 408 |
| **40** | 2-hydroxy-3-methoxybenzylamino | fluoro | C=51,3/51,0; H=4,8/4,8; N=16,6/16,9 | 420 | 422 |
| **41** | 2-hydroxy-5-methoxybenzylamino | fluoro | C=51,3/50,8; H=4,8/4,8; N=16,6/17,0 | 420 | 422 |
| **42** | 2-hydroxy-3-methylbenzylamino | fluoro | C=54,4/54,7; H=5,3/5,2; N=16,7/16,4 | 418 | 420 |
| **43** | 2-hydroxy-5-methylbenzylamino | fluoro | C=54,4/54,2; H=5,3/5,3; N=16,7/16,9 | 418 | 420 |
| **44** | 2-hydroxy-3-chlorobenzylamino | fluoro | C=48,0/47,7; H=4,0/5,3; N=16,5/16,8 | 424 | 426 |
| **45** | 2-methoxy-3-hydroxybenzylamino | fluoro | C=51,3/50,9; H=1,8/4,7; N=16,6/17,0 | 420 | 422 |
| **46** | 2,4-dimethylbenzylamino | fluoro | C=56,6/56,3; H=5,5/5,7; N=17,4/17,6 | 402 | 404 |
| **47** | 2-amino-3-chlorobenzylamino | fluoro | C=48, 1/47,6; H=4,3/4,3; N=19,8/20,2 | 423 | 425 |
| **48** | 2-amino-5-chlorobenzylamino | fluoro | C=48,1/47,8; H=4,3/4,2; N=19,8/20,1 | 423 | 425 |
| **49** | 2-hydroxy-5-chlorobenzylamino | fluoro | C=50.8/51.0; H=5.0/5.1; N=16.4/16.7 | 424 | 426 |
| **50** | 2-hydroxy-5-fluorobenzylamino | fluoro | C=52.8/52.9; H=5.2/5.0; N=17.1/17.4 | 408 | 410 |

### EXAMPLE 13

### 2-Amino-6-(2-methoxybenzylamino)purine riboside

To a suspension of 2-amino-6-chloropurine riboside (1.69g; 0.01mol) and 2-methoxybenzylamine (1.23g; 0.01mol) in *n*-butanol (20mL) triethylamine (3.5mL; 0.025mol) was added. The resulting thick suspension was stirred at 110°C for 3 hours The TLC showed the absence of starting material and the presence of desired product. The reaction mixture was then cooled to room temperature.The white precipitate was filtered off, rinsed with *n*-butanol (3x10mL) and water (3x10mL) and dried in the drying oven into constant weight. Yield: 2.19g (85%). The crude product was purrified as hydrochloride salt by crystallization from 2.5M methanolic hydrogen chloride to give 2.17g of almost white crystals (as hydrochloride). TLC (chloroform-methanol-NH₄OH; 4:1:0.05): one single spot, free of starting material. HPLC purity: 99+%.

**Table 3: Compounds Prepared by the Method of Example 13**

| SUBSTITUENT | | | CHN ANALYSES | MS ANALYSES-ZMD | |
|---|---|---|---|---|---|
| | R6 | R2 | [%] | [M-H]⁻ a) | [M+H]⁺b) |
| **51** | 2,3-dimethoxybenzylamino | amino | C=52,8/52,7; H=5,6/5,7; N=19,4/19,7 | 431 | 433 |
| **52** | 2,4-dimethoxybenzylamino | amino | C=52,8/52,5; H=5,6/5,6; N=19,4/19,8 | 431 | 433 |
| **53** | 2,4,5-trimethoxybenzylamino | amino | C=51,9/51,6; H=5,7/5,6; N=18,2/18,5 | 461 | 463 |
| **54** | 2,6-dimethoxy-4-hydroxybenzylamino | amino | C=50,9/50,5; H=5,4/5,4; N=18,7/19,2 | 447 | 449 |
| **55** | 2,3-dihydroxybenzylamino | amino | C=50,5/50,1; H=5,0/5,0; N=20,8/21,1 | 403 | 405 |
| **56** | 2,5-dihydroxybenzylamino | amino | C=50,5/50,2; H=5,0/5,1; N=20,8/21,3 | 403 | 405 |
| **57** | 2-hydroxy-3-methoxybenzylamino | amino | C=51,7/51,1; H=5,3/5,4; N=20,1/19,4 | 417 | 419 |
| **58** | 2-hydroxy-5-methoxybenzylamino | amino | C=51,7/51,5; H=5,3/5,3; N=20,1/20,3 | 417 | 419 |
| **59** | 2-hydroxy-3-methylbenzylamino | amino | C=53,7/53,4; H=5,5/5,4; N=20,9/21,4 | 401 | 403 |
| **60** | 2-hydroxy-5-methylbenzylamino | amino | C=53,7/53,4; H=5,5/5,5; N=20,9/21,2 | 401 | 403 |
| **61** | 2-hydroxy-3-chlorobenzylamino | amino | C=48,3/48,6; H=4,5/4,4; N=19,9/20,4 | 421 | 423 |
| **62** | 2-methoxy-3-hydroxybenzylamino | amino | C=51,7/51,2; H=5,3/5,5; N=20,1/20,7 | 417 | 419 |
| **63** | 2,4-dimethylbenzylamino | amino | C=57,0/56,7; H=6,0/6,1; N=21,0/21,3 | 399 | 401 |
| **64** | 2-amino-3-chlorobenzylamino | amino | C=48,4/48,7; H=4,8/4,8; N=23,2/23,6 | 420 | 422 |

### EXAMPLE 14

### 2-Mercaptoxamthine

4,5-Diamino-6-hydroxy-2-mercaptopyrimidine (MW 158, Aldrich 95%, used as supplied, 100 g) was refluxed with 90% formic acid (500 mL) for 2 h in a 3 1, 3-neck flask with mechanical stirring. The mixture initially solidified and a further 100 mL of formic acid is added. The mixture was cooled on ice and crude 4-amino-5-formamido-6-hydroxy-2-mercaptopyrimidine was filtered on a large sinter. It was covered with a rubber dam and dried under vacuum for 30 min giving a light-yellow product. The filtration cake was suspended in formamide (225 mL) in a large beaker and heated at 175-185 °C (bath temperature) in a liquid paraffin bath with occasional hand stirring for 2 h. Considerable frothing occurs in the early stages. The mixture was cooled to room temperature and filtered with dam on large sinter, then dissolved in approximatelly 2 L of 1M NaOH and precipitated with glacial AcOH at 10 °C. Then it was filtered, pressed dry and vacuum dried at 95 °C for 2 h. Yield 103 g (97%, cf 85% theory, product incompletely dry).

### EXAMPLE 15

### 2-Benzylmercapto-6-purinol

5.28 g 8-mercaptoxanthine (Mᵣ 168.2) was suspended in 78.5 mL of 1 M NaOH and diluted to approximatelly 400 mL with water. Starting material did not completely dissolve even with the 2.5-fold excess of base. Benzyl chloride (3.7 mL) was added in a single portion and the mixture was vigorously stirred for approximatelly 3 h at room temperature, adjusted to pH 5 with glacial AcOH to yield a red precipitate. The precipitate was filtered, washed thoroughly with water and dried overnight in vacuo at 80 °C to give 7.33 g of a salmon-pink solid which was used without further purification. Yield 95 %, HPLC: 98%.

### EXAMPLE 16

### 2-Benzylmercapto-6-chloropurine

2-Benzylmercaptoxanthine (Mr 258, 27.6 g, 0.107 mmole) was covered with phosphorus oxychloride (506 mL) and *N,N'*-diethylaniline (40 mL) added. The mixture was refluxed for 1.5 h. Excess oxychloride was removed in vacuo to give a syrupy which was poured onto ice (2 kg). Sodium hydroxide was added, with cooling, to dissolve the solid. The mixture was acidified to pH 1 with concentrated HCl to give a solid which was filtered off, washed with water and dried in vacuo at 70 °C for several hours. The crude product was triturated with a small volume of MeOH to yield a yellow solid which was dried in vacuo at 70 °C. Recrystallization from MeOH, BuOH or EtOAc was unsuccessful. Yield 17.5 g (59%). Crude 2-benzylmercapto-6-chloropurine and 2-benzylmercaptoxanthine dissolve readily in DMSO. Column cromatography on silica, 5% MeOH/chloroform, crude 2-benzylmercapto-6-chloropurine shows sharp, major spot 0.39 with 4 medium contaminants at 0.05, 0.14, 0.60, and 0.65. No 2-benzylmercaptoxanthine starting material present (2-benzylmercaptoxanthine standard gives 2 equal spots at 0.10 and 0.18 = tautomers). Column cromatography on silica gel - 100% chloroform 2-benzylmercapto-6-chloropurine: 0.02; with 2.5 % MeOH/chloroform, 2-benzylmercapto-6-chloropurine: 0.25. Collect 36 x 100 mL fractions and locate in the same solvent; gives good decolour. Product elutes 6 to 36 (= final fraction collected, 2-benzylmercapto-6-chloropurine very faint in this fraction); 4 faster-running contaminants 3 to 9. No elution of slower-running contaminants up to 36. Pool 7 to 36 and dry to yellow solid. 2-Benzylmercapto-6-chloropurine is very soluble in 100% MeOH (and 10% MeOH/chloroform); crystallization from 50% aqueous MeOH in fridge to give colourless needles. Product was dried to constant weight over calcium chloride followed by phosphorus pentoxide at 45 °C overnight. Reaction yielded very pale yellow needles (3 g); m.p. 176-178 °C, sharp, no decomposition occured. TLC (silica - 5%MeOH/chloroform). Single, sharp major spot (0.53) at 25, 50 & 100 µg load; 2 faint contaminants (0.76 & 0.83). Purity estimated 95+%.

### EXAMPLE 17

### 2-Benzylmercapto-6-(2-hydroxy-3-methoxybenzylamino)purine

2-Benzylmercapto-6-chloropurine (3.9g), 2-hydroxy-3-methoxybenzylamine (3.42 g) were dissolved in n-BuOH (100 mL) containing triethylamine (7.5 mL) refluxed for 2 h. Reaction mixture was cooled and dried off in vacuo, cooled on ice and washed with water (400 mL). Crude product was left standing overnight in the fridge, filtered to yield a dark-brown solid which did not decolourise on attempted MeOH recrystalization. Dry crude product was poured onto 25 g silica and purified using column chromatography over a further 100 g silica packed in chloroform. Elute with 500 mL 100% chloroform followed by 500 mL x 1.25% MeOH/chloroform then 1.5 L x 2.5% MeOH/chloroform. 100 mL of fractions was collected, locating in 2.5% MeOH/chloroform. Product eluted in fractions 11 to 16 inclusive. Fractions 12 to 15 were evaporated to yield light-brown solid. Pruduct was trituated twice from a small volume of boiling MeOH to remove most of colour and re-crystalize from MeOH with decolourisation in freezer overnight. Yield 1.18 g of virtually white, amorphous solid. TLC (2.5% MeOH/chloroform) shows only a single spot with no contaminants detectable with a range of loadings from 5 to 50 µg, purity 98+%. 95% EtOH/HCl 307 (303); 95% EtOH 291 (288); 95% EtOH/ammonia 298 (293). A further 600 mg of crude yellow product was recovered from the liquors (TLC shows 2 approx. equal spots). 8-Isomer is too soluble in 100% MeOH for succesful ppt.

### EXAMPLE 18

### 2-Benzylmercapto-6-(2-hydroxy-3-methoxybenzylamino)purine riboside

2-Benzylmercapto-6-chloropurine (4 g) was mixed in a 100 mL round bottom flask with β-D-ribofuranose 1,2,3,5-tetraacetate (6.4 g, Aldrich) and fused at 145 to 150 °C (bath temperature) in a liquid paraffin. Iodine (approximately 100 mg) was added and the mixture evaporated on the water pump for 30 min. The black tar was triturated thoroughly with chloroform and filtered to remove a small amount of insoluble black residue. The fusion reaction was repeated a further 3 times and the chloroform extracts pooled and purified by column chromatography on 2 x 400 g Merck type 7734 silica gel packed in 2 L 20% ethyl acetate/toluene. The crude reaction mix was loaded in a total of 100 mL packing solvent and the column eluted with the remaining packing solvent (around 1L) followed by 1L 20% ethyl acetate rising in 2L/20% x 1 L steps to 30% run at fastest gravity flow rate. Discard first 2500 mL and collect 150 mL fractions. Monitor by TLC in ethyl acetate/toluene (1/2, v/v) (starting material = 0.06, product 0.3). Total elution volume approximately 8 l and 5 h (a test of benzene/ethyl acetate: 5/1, v/v). The brown syrup was dried in vacuo and dissolved in a little dry methanol, cooled to 20°C and deacetylated overnight in 1000 mL anhydrous methanol saturated with ammonia at 20 °C. Remove ammonia at room temperature and methanol at 35 °C followed by 10 min at 45 °C to remove acetamide. Recrystallise twice with decolourisation from boiling water. The product crystallised easily into pale yellow needles with only small solubility in water. Air dry, then to constant weight over phosphorus pentoxide. Yield 4.95 g (18.6 %); mp 193-194 °C; HPLC: 25cm ODS 0 - 30% acetonitrile, 1 mL.min⁻¹ over 30 min. Rt: 24.0 min. Overall HPLC purity = 91%. TLC chloroform/methanol (9/1, v/v) shows major spot 0.40.

### EXAMPLE 19

### 2-Mercapto-6-(2-hydroxy-3-methoxybenzylamino)purine riboside

2-Benzylmercapto-6-(2-hydroxy-3-methoxybenzylamino)purine riboside (1.18 g) was dissolved in liquid ammonia (125 mL) to yield a clear yellow solution. No external cooling necessary for handling liquid ammonia. Sodium was added in small portions until the blue colouration persisted for 15 mins. A small amount of solid ammonium chloride was added cautiously to remove excess Na. The ammonia was evaporated to a small volume on a hot plate and ether (125 mL) added (to remove bibenzyl formed as a by-product). After most of the remaining ammonia had been evolved the ether extract was extracted with water (2 x 65 mL). The aqueous extrakt (at pH 12 to 13) was adjusted to 5 with AcOH cooling to below 10 on dry ice. A creamy solid precipitated. The solid was filtered, washed thoroughly with water and dried overnight over calcium chloride to yield a virtually white, very light powder. Yield: 760 mg. 2-Mercapto-6-(2-hydroxy-3-methoxybenzylamino)purine riboside gives a sharp, symmetrical peak on HPLC (300 nm) and moves readily on TLC in MeOH/chloroform mixtures. It is soluble in EtOH. EtOH solution develops strong yellow colour on standing at room temperature for several days.

**Table 4: Compounds Prepared by the Method of Example 19**

| SUBSTITUENT | | | CHN ANALYSES | MS ANALYSES-ZMD | |
|---|---|---|---|---|---|
| | **R6** | **R2** | [%] | [M-H]⁻ a) | [M+H]⁺ b) |
| **65** | 2,3-dimethoxybenzylamino | mercapto | C=50,8/50,7; H=5,2/5,5; N=15,6/15,4 | 448 | 450 |
| **66** | 2,4-dimethoxybenzylamino | mercapto | C=50,8/50,1; H=5,2/5,3; N=15,6/15,9 | 448 | 450 |
| **67** | 2,4,5-trimethoxybenzylamino | mercapto | C=50,1/49,6; H=5,3/5,3; N=14,6/15,0 | 478 | 480 |
| **68** | 2,6-dimethoxy-4-hydroxybenzylamino | mercapto | C=49,0/49,5; H=5,0/5,2; N=15,1/14,6 | 464 | 466 |
| **69** | 2,3-dihydroxybenzylamino | mercapto | C=48,5/48,2; H=4,5/4,4; N=16,6/16,3 | 420 | 422 |
| **70** | 2,5-dihydroxybenzylamino | mercapto | C=48,5/48,0; H=4,5/4,6; N=16,6/16,9 | 420 | 422 |
| **71** | 2-hydroxy-3-methoxybenzylamino | mercapto | C=49,6 /49,4; H=4,9/4,8; N=16,1/15,9 | 434 | 436 |
| **72** | 2-hydroxy-5-methoxybenzylamino | mercapto | C=49,6/49,2; H=4,9/4,6; N=16,1/15,7 | 434 | 436 |
| **73** | 2-hydroxy-3-methylbenzylamino | mercapto | C=51,5/51,1;H=5,0/5,1; N=16,7/16,2 | 418 | 420 |
| **74** | 2-hydroxy-5-methylbenzylamino | mercapto | C=51,5/50,9; H=5,0/4,9; N=16,7/16,9 | 418 | 420 |
| **75** | 2-hydroxy-3-chlorobenzylamino | mercapto | C=46,4/45,9; H=4,1/4,3; N=15,9/16,4 | 438 | 440 |
| **76** | 2-methoxy-3-hydroxybenzylamino | mercapto | C=49,6/49,1; H=4,9/4,5; N=16,1/16,8 | 434 | 436 |
| **77** | 2,4-dimethylbenzylamino | mercapto | C=54,7,6/54,2; H=5,6/5,5; N=16,8/17,3 | 418 | 420 |
| **78** | 2-amino-3-chlorobenzylamino | mercapto | C=46,5/46,0; H=4,4/4,3; N=19,2/19,7 | 437 | 439 |
| **79** | 2-chloro-5-methoxybenzylamino | mercapto | C=47,6/47,2; H=4,4/4,5; N=15,4/15,9 | 452 | 454 |
| **80** | 2-methoxy-3-chlorobenzylamino | mercapto | C=47,6/47,2; H=4,4/4,5; N=15,4/15,7 | 452 | 454 |

### EXAMPLE 20

### 2-Methylmercaptoxanthine

2-Mercaptoxanthine (103g, 0.613 mol) was dissolved in 2M NaOH (613 mL) and water (245 mL) in a 3L, 3-neck flask accompanied with mechanical stirrer and removable ice-bath. Dimethyl sulphate was added (77 g, 58 mL) dropwise, keeping temperature between 25 - 40 °C using ice-bath as necessary. Reaction mixture was stirred for 1 h and stood at room temperature overnight. The dark-red liquid was filtered and the precipitated product was acidified with glacial acetic acid to pH 5 and 10. Solid was filtered on 24 cm Büchner and recrystallised from boiling water (approx.1500 mL) with decolourisation. The substance was crystallised at 10 °C. Filtered and vacuum dried at 95 °C for 3 h, then driedto constant weight over phosphorus pentoxideThe mother liquor reduction to half volume yielded further product. Yield 1^{st} crop 48 g, 2nd crop 16 g. Total 48% from diamino-hydroxy mercaptopyrimidine starting material. UV purity 80% calculated as 1.5 hydrate. UV pH1: 266.5 (265); pH11: 271 (270).

### EXAMPLE 21

### 2-Methylmercapto-6-chloropurine

2-Methylmercaptoxanthine (65 g, recrystallised from water, light-yellow powder, dried to constant weight over phosphorus pentoxide) was placed in a 3 l, 3-neck flask and covered with phosphorus oxychloride (975 mL) and *N,N*'-diethylaniline (97.5 mL). The mixture was refluxed with mechanical stirring for 1.5 h. Virtually all the excess phosphorus oxychloride was removed in vacuo (dry evaporator, on water pump) at 55-60 °C and the residue poured onto ice (0 °C, 1.75 kg) in a 10 l flask with hand-stirring. The mixture was stirred for 10 min to complete hydrolysis of the oxychloride and extracted with ethyl acetate (4 x 2.5 L). The combined ethyl acetate extracts were washed with water (3 x 1 L) and dried to a dark solid. The crude solid was recrystallised with decolourisation from ethanol and to constant weight over phosphorus pentoxide. Yield 23.6 g (33%). UV purity 99% HPLC (25 x 0.4 cm ODS, 0 to 30% acetonitrile, 1 mL/min, 30 min), Rt: 26.8 min, purity 92%. TLC (chloroform/methanol, 9/1, v/v) Rf 0.80. Remaining stock further purified: dissolved with stirring in minimum amount of 2 M NaOH, filtered, cooled and precipitated with glacial AcOH at pH 5 overnight at 10 °C. 3 x Re-crystallisation from ethanol with prolonged (30 min) boiling with substantial amount of charcoal provided good decolourisation. Yield approx. 7 g of lemon-yellow, microcrystalline solid. HPLC (25 x 0.46 cm ODS-2 Spherisorb 5µm, 0-40% acetonitrile, 30 min, 2 mL/min), Rt 7.8 min, purity: 99%.

### EXAMPLE 22

### 2-Methylmercapto-6-chloro-9-B-D-ribofuranosylpurine

2-Methylmercapto-6-chloropurine (4 g dried to constant weight over phosphorus pentoxide) was mixed in a 100 mL round bottom flask with β-D-ribofuranose 1,2,3,5-tetraacetate (6.4 g, Aldrich) and fused at 145 - 150 °C (bath temperature) in a liquid paraffin. Iodine (approximately 100 mg) was added and the mixture was evacuated for 30 min. The black tar was triturated thoroughly with chloroform and filtered to remove a small amount of insoluble black residue. The fusion reaction was repeated three times and the chloroform extracts pooled and purified by column chromatography on 2 x 400 g Merck type 7734 silica gel packed in 2 L 20% ethyl acetate/toluene. The crude reaction mixture was loaded in a total of 100 mL packing solvent and the column was eluted with the remaining packing solvent (around 1L) followed by 1L 20% ethyl acetate rising in 2L/20% x 1L steps to 30% run at fastest gravity flow rate - first 2500 mL were discarted and 150 mL of fractions was collected. Fractions were monitored by TLC in ethyl acetate/toluene (1/2, v/v) (starting material = 0.06, product 0.25). Total elution volume was approximately 8 l and the time of elution was 5 h (a test of benzene/ethyl acetate: 5/1, v/v). The major product, the B-isomer, indicated a yield of B-triacetate of 26 g (71%). The brown syrupy was dried in vacuo and dissolved in dry methanol, cooled to 20 °C and deacetylated overnight in 1000 mL of anhydrous methanol saturated with ammonia at 20 °C. The ammonia was removed at room temperature and methanol at 35 °C that was followed by 10 min at 45 °C to remove acetamide. The product was recrystallised twice with decolourisation from boiling water (more satisfactory than ethanol). The compound easily crystallised to yield pale yellow needles with only small loss in water. The product was dried on air, then to constant weight over phosphorus pentoxide. Yield 4.95 g (18.6 %); mp 187-188 °C; HPLC: 25cm ODS 0-30% acetonitrile, 1 mL per min over 30 min. Rt: B-anomer 26.0 min, A-anomer 26.5 min. Twice recrystallised product showed some material at 20.6 min (not 2-methylthio-6-chloropurine starting material, tR 26.8 min) but no A-anomer. Overall HPLC purity = 91%. TLC chloroform/methanol (9/1, v/v) showed major spot 0.38 + 3 faint slower moving contaminants at 0.31, 0.19 and 0.09. TLC ethyl acetate/ethanol (10/1, v/v) shows major spot at 0.38 + 2 faint contaminants at 0.11 and 0.02. Expect B-anomer 0.31, A-anomer 0.19. MW free riboside: 332.8 C₁₁H₁₃O₄N₄SCl, MW triacetate: 459.

### EXAMPLE 23

### 2-Methylmercapto-6-(2-hydroxy-3-methoxybenzylamino)purine riboside

2-Methylmercapto-6-chloropurine riboside (3.3 g, 10 mmol) was refluxed with 2-hydroxy-3-methoxybenzylamine (7.7 g, 12.5 mmol) in butanol (100 mL) containing triethylamine (3.2 mL) for 2 h. The butanol was removed under vacuum at 50 to 60 °C. Purification of the mixture directly by trituration with water was unsuccessful. Crude reaction mixture was purified by CC over 100 g silica packed in 5% methanol/chloroform and eluted with a methanol/chloroform gradient (5 to 20% in 2.5% x 250 mL steps, collecting 50 mL samples, discarding the first 500 mL). Product elutes fractions 19 to 30. TLC (15% methanol/chloroform) showed several faster eluting compounds as well as some starting material. Final product was contaminated with major slower running component. Dry to almost white powder and purify by column chromatography over 100 g of silica packed and eluted with ethyl acetate/ethanol (10/1, v/v), collecting 25 mL fractions. The repeat column chromatography purification gives rapid elution with good decolourisation. Yield: 2.2 g (56%) white powder. HPLC shows 1 contaminant remaining, overall purity of product 97 %. Recrystalization from ethyl acetate gave brilliant white, microcrystalline solid. Product is very soluble in methanol and water but recrystallises well from ethyl acetate.

**Table 5: Compounds Prepared by the Method of Example 23**

| SUBSTITUENT | | | CHN ANALYSES | MS ANALYSES-ZMD | |
|---|---|---|---|---|---|
| | R6 | R2 | [%] | [M-H]⁻ a) | [M+H]⁺ b) |
| **81** | 2,3-dimethoxybenzylamino | methylmercapto | C=51,8 /51,2; H=5,4/5,5; N=15,1/15,6 | 462 | 464 |
| **82** | 2,4-dimethoxybenzylamino | methylmercapto | C=51,8/51,5; H=5,4/5,4; N=15,1/15,4 | 462 | 464 |
| **83** | 2,4,5-trimethoxybenzylamino | methylmercapto | C=51,1 /51,4; H=5,5/5,6; N=14,2/14,7 | 492 | 494 |
| **84** | 2,6-dimethoxy-4-hydroxybenzylamino | methylmercapto | C=50,1/51,0; H=5,3/5,5; N=14,6/15,1 | 478 | 480 |
| **85** | 2,3-dihydroxybenzylamino | methylmercapto | C=49,6 /50,2; H=4,9/4,8; N=16,1/16,2 | 434 | 436 |
| **86** | 2,5-dihydroxybenzylamino | methylmercapto | C=49,6/50,0; H=4,9/5,1; N=16,1/15,4 | 434 | 436 |
| **87** | 2-hydroxy-3-methoxybenzylamino | methylmercapto | C=50,8/50,3; H=5,2/5,0; N=15,6/16,1 | 448 | 450 |
| **88** | 2-hydroxy-5-methoxybenzylamino | methylmercapto | C=50,8/50,4; H=5,2/5,1; N=15,6/16,2 | 448 | 450 |
| **89** | 2-hydroxy-3-methylbenzylamino | methylmercapto | C=52,7/52,2; H=5,4/5,4; N=16,2/16,7 | 432 | 434 |
| **90** | 2-hydroxy-3-methylbenzylamino | methylmercapto | C=52,7/52,3; H=5,4/5,5; N=16,2/16,6 | 432 | 434 |
| **91** | 2-hydroxy-3-chlorobenzylamino | methylmercapto | C=47,6 /47,2; H=4,4/4,3; N=15,4/15,9 | 452 | 454 |
| **92** | 2-methoxy-3-hydroxybenzylamino | methylmercapto | C=50,8/50,1; H=5,2/4,9; N=15,6/16,3 | 448 | 450 |
| **93** | 2,4-dimethylbenzylamino | methylmercapto | C=55,7/55,0; H=5,8/5,7; N=16,2/16,8 | 430 | 432 |
| **94** | 2-amino-3-chlorobenzylamino | methylmercapto | C=47,7/47,1; H=4,7/5,0; N=18,6/19,3 | 451 | 453 |
| **95** | 2-chloro-5-methoxybenzylamino | methylmercapto | C=48,8 /48,2; H=4,7/4,7; N=15,0/14,7 | 466 | 468 |
| **96** | 2-methoxy-3-chlorobenzylamino | methylmercapto | C=48,8/48,4; H=4,7/4,6; N=15,0/15,3 | 466 | 468 |
| **97** | 2,4-difluorobenzylamino | methylmercapto | C=51.9/51.9;H=5.3/5.1; N=15.7/15.8 | 438 | 440 |

### EXAMPLE 24

### 2-Methyl-6-(3-methoxybenzylamino)purine riboside

Triethylamine (3.5 mL; 0.025 mol) was added to a suspension of 2-methyl-6-chloropurine riboside (1.69 g; 0.01 mol) and 3-methoxybenzylamine (1.23 g; 0.01 mol) in *n*-butanol (20 mL). The resulting thick suspension was stirred at 110 °C for 3 hours. The TLC showed the absence of starting material and the presence of desired product. The reaction mixture was then cooled to room temperature. The white precipitate was filtered off, rinsed with *n*-butanol (2x10mL) and water (3x10mL) and dried in the drying oven into constant weight. Yield: 1.92 g (75 %). The crude product was purrified as hydrochloride salt by crystallization from 2.5M methanolic hydrogen chloride to give almost white crystals (as hydrochloride). TLC (chloroform-methanol-NH₄OH; 4:1:0.05): one single spot, free of starting material. HPLC purity: 99+%

**Table 6: Compounds Prepared by the Method of Example 24**

| SUBSTITUENT | | | CHN ANALYSES | MS ANALYSES-ZMD | |
|---|---|---|---|---|---|
| | R6 | R2 | [%] | [M-H]⁻ a) | [M+H]⁺ b) |
| **98** | 2,3-dimethoxybenzylamino | methyl | C=55,7 /55,3; H=5,8/5,7; N=16,2/16,9 | 430 | 432 |
| **99** | 2,4-dimethoxybenzylamino | methyl | C=55,7 /55,4; H=5,8/5,8; N=16,2/16,7 | 430 | 432 |
| **100** | 2,4,5-trimethoxybenzylamino | methyl | C=54,7 /54,2; H=5,9/6,0; N=15,2/15,9 | 460 | 462 |
| **101** | 2,6-dimethoxy-4-hydroxybenzylamino | methyl | C=55,7/55,4; H=5,8/5,8; N=15,6/16,1 | 446 | 448 |
| **102** | 2,3-dihydroxybenzylamino | methyl | C=53,6 /53,2; H=5,3/5,2; N=17,4/18,0 | 404 | 404 |
| **103** | 2,5-dihydroxybenzylamino | methyl | C=53,6 /53,4; H=5,3/5,4; N=17,4/17,7 | 404 | 404 |
| **104** | 2-hydroxy-3-methoxybenzylamino | methyl | C=54,7/54,1; H=5,6/5,5; N=16,8/17,3 | 416 | 418 |
| **105** | 2-hydroxy-5-methoxybenzylamino | methyl | C=54,7 /54,4; H=5,6/5,6; N=16,8/17,2 | 416 | 418 |
| **106** | 2-hydroxy-3-methylbenzylamino | methyl | C=56,9 /56,5; H=5,8/5,8; N=17,5/17,9 | 400 | 402 |
| **107** | 2-hydroxy-5-methylbenzylamino | methyl | C=56,9 /56,4; H=5,8/5,7; N=17,5/17,7 | 400 | 402 |
| **108** | 2-hydroxy-3-chlorobenzylamino | methyl | C=51,3/50,9; H=4,8/4,7; N=16,6/17,1 | 420 | 422 |
| **109** | 2-methoxy-3-hydroxybenzylamino | methyl | C=54,7/54,9; H=5,6/5,4; N=16,8/16,3 | 416 | 418 |
| **110** | 2,4-dimethylbenzylamino | methyl | C=60,1/59,6; H=6,3/6,1; N=17,5/18,0 | 400 | 402 |
| **111** | 2-amino-3-chlorobenzylamino | methyl | C=51,4/50,9; H=5,1/5,0; N=20,0/20,7 | 400 | 402 |
| **112** | 2-chloro-5-methoxybenzylamino | methyl | C=52,4/51,5; H=5,1/5,3; N=16,1/16,9 | 400 | 402 |
| **113** | 2-methoxy-3-chlorobenzylamino | methyl | C=52,4/51,7; H=5,1/5,2; N=16,1/17.0 | 400 | 402 |

### EXAMPLE 25

### 2-Hydroxyethylamino-6-(2-hydroxy-3-methoxybenzylamino)purine riboside

Triethylamine (3.5 mL; 0.025 mol) was added to a suspension of 2-methyl-6-chloropurine riboside (1.69 g; 0.01 mol) and 2-hydroxy-3-methoxybenzylamine (1.23 g; 0.01 mol) in *n*-butanol (20mL). The resulting thick suspension was stirred at 110°C for 3 hours The TLC showed the absence of starting material and the presence of desired product. The reaction mixture was then cooled to room temperature.The white precipitate was filtered off, rinsed with n-butanol (2x10mL) and water (3x10mL) and dried in the drying oven into constant weight. Yield: 1.92g (75%). The crude product was purrified as hydrochloride salt by crystallization from 2.5M methanolic hydrogen chloride to give almost white crystals (as hydrochloride). TLC (chloroform-methanol-NH₄OH; 4:1:0.05): one single spot, free of starting material. HPLC purity: 99+%

**Table 7: Compounds Prepared by the Method of Example 25**

| SUBSTITUENT | | | CHN ANALYSES | MS ANALYSES-ZMD | |
|---|---|---|---|---|---|
| | R6 | R2 | [%] | [M-H]⁻ a) | [M+H]⁺ b) |
| **114** | 2-hydroxy-3-methoxybenzylamino | 2-hydroxyethylamino | C=51,9/51,4; H=5,7/6,2; N=18,2/18,5 | 461 | 463 |
| **115** | 2-hydroxy-3-methoxybenzylamino | 2-hydroxypropylamino | C=52,9 /52,3; H=5,9/6,2; N=17,6/17,9 | 475 | 477 |
| **116** | 2-hydroxy-3-methoxybenzylamino | 3-hydroxypropylamino | C=52,9/53,5; H=5,9/6,4; N=17,6/18,0 | 475 | 477 |
| **117** | 2-hydroxy-3-methoxybenzylamino | 2-aminoethylamino | C=52,1/51,4; H=5,9/6,7; N=21,3/20,8 | 460 | 462 |
| **118** | 2-hydroxy-3-methoxybenzylamino | 2-aminopropylamino | C=53,1/53,6; H=6,3/6,4; N=20,6/20,8 | 476 | 476 |
| **119** | 2-hydroxy-3-methoxybenzylamino | 3-aminopropylamino | C=53,1/52,4; H=6,3/6,0; N=20,6/19,8 | 476 | 476 |
| **120** | 2-hydroxy-3-methoxybenzylamino | (R)-1-(hydroxymethyl)-propylamino | C=53,9/53,6; H=6,2/6,8; N=17,1/17,0 | 489 | 491 |
| **121** | 2-hydroxy-3-methoxybenzylamino | [bis-(2-hydroxyethyl)]-amino | C=52,2/52,7; H=6,0/6,2; N=16,6/17,1 | 505 | 507 |
| **122** | 2-hydroxy-3-methoxybenzylamino | (R)-1-isopropyl-2-hydroxyethylamino) | C=54,8/54,1; H=6,4/6,8; N=16,7/17,0 | 503 | 505 |
| **123** | 2-hydroxy-3-methoxybenzylamino | 4-aminocyclohexylamino | C=55,9/55,3; H=6,5/6,7; N=19,0/18,6 | 514 | 516 |
| **124** | 2-hydroxy-3-methoxybenzylamino | 2-aminocyclohexylamino | C=55,9/55,1; H=6,5/6,5; N=19,0/18,7 | 514 | 516 |
| **125** | 2-hydroxy-5-methoxybenzylamino | 2-hydroxyethylamino | C=51,9/52,7; H=5,7/5,2; N=18,2/18,4 | 461 | 463 |
| **126** | 2-hydroxy-5-methoxybenzylamino | 2-hydroxypropylamino | C=52,9 /53,5; H=5,9/5,3; N=17,6/16,9 | 475 | 477 |
| **127** | 2-hydroxy-5-methoxybenzylamino | 3-hydroxypropylamino | C=52,9/52,3; H=5,9/5,6; N=17,6/17,0 | 475 | 477 |
| **128** | 2-hydroxy-5-methoxybenzylamino | 2-aminoethylamino | C=52,1/52,7; H=5,9/6,0; N=21,3/21,1 | 460 | 462 |
| **129** | 2-hydroxy-5-methoxybenzylamino | 2-aminopropylamino | C=53,1/53,7; H=6,3/6,5; N=20,6/19,9 | 476 | 476 |
| **130** | 2-hydroxy-5-methoxybenzylamino | 3-aminopropylamino | C=53,1/53,3; H=6,3/5,9; N=20,6/21,1 | 476 | 476 |
| **131** | 2-hydroxy-5-methoxybenzylamino | (R)-1-(hydroxymethyl)-propylamino | C=53,9/53,2; H=6,2/6,4; N=17,1/16,6 | 489 | 491 |
| **132** | 2-hydroxy-5-methoxybenzylamino | [bis-(2-hydroxyethyl)]-amino | C=52,2/51,5; H=6,0/6,3; N=16,6/16,4 | 505 | 507 |
| **133** | 2-hydroxy-5-methoxybenzylamino | (R)-1-isopropyl-2-hydroxyethylamino) | C=54,8/54,4; H=6,4/6,5; N=16,7/16,1 | 503 | 505 |
| **134** | 2-hydroxy-5-methoxybenzylamino | 4-aminocyclohexylamino | C=55,9/56,1; H=6,5/6,5; N=19,0/19,1 | 514 | 516 |
| **135** | 2-hydroxy-5-methoxybenzylamino | 2-aminocyclohexylamino | C=55,9/55,5; H=6,5/6,4; N=19,0/18,1 | 514 | 516 |
| **136** | 2-hydroxy-3-methylbenzylamino | 2-hydroxyethylamino | C=53,8/53,1; H=5,9/5,6; N=18,1/18,7 | 445 | 447 |
| **137** | 2-hydroxy-3methylbenzylamino | 2-hydroxypropylamino | C=53,9/53,2; H=6,2/6,4; N=18,3/18,9 | 459 | 461 |
| **138** | 2-hydroxy-3-methylbenzylamino | 2-aminopropylamino | C=54,9/54,3; H=6,4/6,3; N=21,3/21,1 | 458 | 460 |
| **139** | 2-hydroxy-3-methylbenzylamino | (R)-1-isopropyl-2-hydroxyethylamino) | C=56,6/57,5; H=6,6/6,7; N=17,2/16,7 | 487 | 489 |
| **140** | 2-hydroxy-3-methylbenzylamino | 4-aminocyclohexylamino | C=57,7/57,9; H=6,7/6,2; N=19,6/19,1 | 498 | 500 |
| **141** | 2-hydroxy-5-methylbenzylamino | 2-hydroxyethylamino | C=53,8/53,5; H=5,9/5,8; N=18,1/18,9 | 445 | 447 |
| **142** | 2-hydroxy-5-methylbenzylamino | 2-hydroxypropylamino | C=53,9/53,4; H=6,2/6,9; N=18,3/18,1 | 459 | 461 |
| **143** | 2-hydroxy-5-methylbenzylamino | 2-aminopropylamino | C=54,9/55,5; H=6,4/6,1; N=21,322,0 | 458 | 460 |
| **144** | 2-hydroxy-5-methylbenzylamino | (R)-1-isopropyl-2-hydroxyethylamino) | C=56,6/57,0; H=6,6/6,3; N=17,2/17,6 | 487 | 489 |
| **145** | 2-hydroxy-5-methylbenzylamino | 4-aminocyclohexylamino | C=57,7/57,1; H=6,7/7,0; N=19,6/20,2 | 498 | 500 |

### EXAMPLE 26

### Stimulatory effect of novel compounds on plant cell division

Cytokinin-dependent tobacco callus *Nicotiana tabacum* L. cv. Wisconsin 38 was maintained at 25 °C in darkness on modified MS medium, containing per 1 liter: 4 µmol of nicotinic acid, 2.4 µmol of pyridoxine hydrochloride, 1.2 µmol of thiamine, 26.6 µmol of glycine, 1.37 µmol of glutamine, 1.8 µmol of myo-inositol, 30 g of sucrose, 8 g of agar, 5.37 µmol of NAA and 0.5 µmol of the compound tested. Subcultivation was carried out every three weeks. Fourteen days before the bioassay, the callus tissue was transferred to the media without the compound tested. The biological activity was determined from the increase of the fresh callus weight after four weeks of cultivation. Five replicates were prepared for each concentration of the compound tested and the entire test was repeated twice. From the obtained data, the concentration with the highest activity was selected for each compound tested. Relative activity of the compound at this concentration was calculated (Table 8).

The compounds to be tested were dissolved in dimethylsulfoxide (DMSO) and the solution brought up to 10⁻³ M with distilled water. This stock solution was further diluted with the respective media used for the biotest to a concentration ranging from 10⁻⁸ M to 10⁻⁴ M. The final concentration of DMSO did not exceed 0.2 % and therefore did not affect the biological activity in the assay system used.

The compounds listed in Table 8 are compared to a "classical cytokinin" kinetin riboside (compound known in the prior art - C = control). The results in Table 8 show that the substitution in position 2 and 6 of the purine riboside ring generally led to an increase of the cytokinin activity in the callus bioassay in comparison to the classical cytokinin analogue.

**Table 8: The effect of novel compounds on the growth of cytokinin-dependent tobacco callus Nicotiana tabacum L. cv. Wisconsins 38**

| No. | Tested compound | | concentration with highest activity (mol.l⁻¹) | activity (%) [10⁻⁵mol.l⁻¹ C = 100%] |
|---|---|---|---|---|
| | R6 | R2 | | |
| **C** | furfurylamino (kinetin) riboside | | 10⁻⁵ | 100 |
| **32** | 2,4-difluorobenzylamino | chloro | 10⁻⁶ | 109.7 (±12) |
| **30** | 2-hydroxy-5-fluorobenzylamino | chloro | 10⁻⁶ | 108.6 (±3) |
| **33** | 2,3,6-trifluorobenzylamino | chloro | 10⁻⁶ | 105.1 (±2) |
| **49** | 2-hydroxy-5-chlorobenzylamino | fluoro | 10⁻⁶ | 112 (±8) |
| **97** | 2,4-difluorobenzylamino | methylmercapto | 10⁻⁶ | 107 (±8) |

### EXAMPLE 27

### In vitro cytotoxic activity of novel compounds

Low cytotoxicity of the compounds is the major property determining the cosmetic use. One of the parameters used, as the basis for cytotoxicity assays, is the metabolic activity of viable cells. For example, a microtiter assay, which uses the Calcein AM, is now widely used to quantitate cell proliferation and cytotoxicity. For instance, this assay is used in drug screening programs and in chemosensitivity testing. Because only metabolically active cells cleave Calcein AM, these assays detect viable cells exclusively. The quantity of reduced Calcein AM corresponds to the number of vital cells in the culture.

Human T-lymphoblastic leukemia cell line CEM; promyelocytic HL-60 and monocytic U937 leukemias; breast carcinoma cell lines MCF-7, BT549, MDA-MB-231; glioblastoma U87MG cells; cervical carcinoma cells HELA; sarcoma cells U2OS and Saos2; hepatocellular carcinoma HepG2; mouse fibroblasts NIH3T3; mouse immortalized bone marrow macrophages B2.4 and B10A.4; P388D1 and L1210 leukemia; B16 and B16F10 melanomas; human osteosarcoma HOS; human myeloid leukemia K-562; human skin melanoma G-361 were used for routine screening of compounds. The cells were maintained in Nunc/Corning 80 cm² plastic tissue culture flasks and cultured in cell culture medium (DMEM with 5 g/l glucose, 2 mM glutamine, 100 U/mL penicillin, 100 µg/mL streptomycin, 10% fetal calf serum and sodium bicarbonate).

The cell suspensions that were prepared and diluted according to the particular cell type and the expected target cell density (2.500-30.000 cells per well based on cell growth characteristics) were added by pipette (80 µl) into 96/well microtiter plates. Inoculates were allowed a pre-incubation period of 24 hours at 37 °C and 5% CO₂ for stabilisation. Four-fold dilutions of the intended test concentration were added at time zero in 20 µl aliquots to the microtiter plate wells. Usually, the compound tested was evaluated at six 4-fold dilutions. In routine testing, the highest well concentration was 100 µM, but it can be the matter of change dependent on the agent. All drug concentrations were examined in duplicates. Incubations of cells with the tested compounds lasted for 72 hours at 37 °C, in 5% CO₂ atmosphere and 100% humidity. At the end of the incubation period, the cells were assayed by using Calcein AM. Ten microliters of the stock solution were pipetted into each well and incubated for 1 hour. Fluorescence (FD) was measured with the Labsystem FIA Reader Fluoroscan Ascent (UK). The tumour cell survival (GI₅₀) was calculated using the following equitation: TCS=(FD_{drug} exposed _{well} / mean FD_{control wells}) x 100%. The GI₅₀ value, the drug concentration lethal to 50 % of the tumour cells, was calculated from the obtained dose response curves.

Zero cytotoxicity of the novel compounds is the basic prerequisite for agricultural and tumor-unrelated applications. The cytoxicity of the novel compounds was tested on a panel of cell lines of different histogenetic and species origin (Table 9). We show herein that equal activities were found in all tumour cell lines tested, however, the non-malignant cells, e.g. NIH3T3 fibroblasts and normal human lymphocytes, were resistant to 2,6-disubstituted purine ribosides induced cytotoxicity. The compounds listed in Tab. 9 can be divided into 2 groups. The first group contains "classical cytokinins" represented by 6-substituted purine ribosides (which are known in the prior art). The second group contains the novel 2,6-disubstituted derivatives of these compounds. The results show that the substitution in position 2 of the purine riboside skeleton generally led to a decrease in the cytotoxic activity in comparison to the "classical cytokinin" analogues. GI50 for NIH3T3 fibroblasts and normal human lymphocytes was always higher than 100 µM. The novel derivatives show no toxicity to normal and tumour cells in concentrations about 100 µM and thus are more suitable for agricultural applications than "classical cytokinins" (6-substituted purine riboside derivatives).

**Table 9: Cytotoxicity of novel compounds for different cancer cell lines**

| | Tested compound | | Cancer cell line / GI50 (µmol/L) | | | | | |
|---|---|---|---|---|---|---|---|---|
| No. | R2 | R6 | K-562 | MCF7 | G-361 | HOS | CEM | HL60 |
| | | 2-hydroxy-5-fluorobenzylamino | >100 | >100 | >100 | >100 | 52 | 24 |
| 30 | Cl | 2-hydroxy-5-fluorobenzylamino | >100 | >100 | >100 | >100 | >100 | >100 |
| | | 2-hydroxy-5-chlorobenzylamino | 36 | 32 | >100 | >100 | 2.4 | 1.6 |
| 29 | Cl | 2-hydroxy-5-chlorobenzylamino | >100 | >100 | >100 | >100 | >100 | >100 |
| | | 2-hydroxy-3-methoxybenzylamino | >100 | >100 | >100 | >100 | 40 | 9.5 |
| 10 | Cl | 2-hydroxy-3-methoxybenzylamino | >100 | >100 | >100 | >100 | >100 | >100 |
| | | 2-hydroxy-4-methoxybenzylamino | 28 | 20 | >100 | >100 | 0.3 | 0.15 |
| 11 | Cl | 2-hydroxy-4-methoxybenzylamino | >100 | >100 | >100 | >100 | >100 | >100 |
| | | 2-hydroxy-5-methylbenzytamino | 18 | 6.9 | >100 | >100 | 0.9 | 10 |
| 13 | Cl | 2-hydroxy-5-methylbenzylamino | >100 | >100 | >100 | >100 | >100 | >100 |
| | | 2-methoxy-3-hydroxybenzylamino | 16.5 | >100 | >100 | >100 | 1.9 | 10 |
| 21 | Cl | 2-methoxy-3-hydroxybenzylamino | >100 | >100 | >100 | >100 | >100 | >100 |

### EXAMPLE 28

### Anti-inflammatory activity of new 2- substituted 6-benzylaminopurine ribosides

Anti-inflammatory activity of several of the 2-substituted 6-benzylaminopurine riboside derivatives of this invention was determined; kinetin riboside was also evaluated as a control. Rat C6 glioma (ATCC No. CCL107) was cultivated in monolayer in serum-free chemically defined medium containing Ham's F10/minimal essential medium (1:1 v/v), 2 mM L-glutamine, 1 % (v/v) minimal essential medium vitamins (100x), 1 % (v/v) minimal essential medium nonessential amino acids (100x), 100 U/mL penicillin, 100 mg/mL streptomycin, and 30 nM sodium selenite. Incubation was performed at 37 °C in a humidified atmosphere. The assays were performed in the logaritmic growth phase at a density of 2.5x10⁵ cells/cm². Intracellular cAMP synthesis was induced by addition of 5 mM (-)-isoproterenol; various amounts of test compounds were added at the same time as the (-)-isoproterenol. After 30 min incubation at 37 °C, the medium was removed and the cellular amount of cAMP was determined using the cAMP-enzyme immunoassay kit from Amersham. The I₅₀ value was determined from a dose-response curve in duplicate. The effect of nine purine analogues was measured after simultaneous addition with isoproterenol.

**Table 10. Modulation of activity of β-adrenergic receptors, 2-substituted derivatives of 6-(substituted benzylamino)purine ribosides**

| No. | R2 | R6 | Effect | I₅₀ (µM) |
|---|---|---|---|---|
| control | - | furfurylamino | none | 0 |
| 10 | chloro | 2-hydroxy-3-methoxybenzylamino | inhibition | 7.5±1 |
| 13 | chloro | 2-hydroxy-5-methylbenzylamino | inhibition | 18.7±4 |
| 30 | chloro | 2-hydroxy-5-fluorobenzylamino | inhibition | 15.7±4 |
| 29 | chloro | 2-hydroxy-5-chlorobenzylamino | inhibition | 12.4±2 |
| 18 | chloro | 2-hydroxy-5-aminobenzylamino | inhibition | 22.7±3 |
| 43 | fluoro | 2-hydroxy-5-methylbenzylamino | inhibition | 20.8±4 |
| 40 | fluoro | 2-hydroxy-3-methoxybenzylamino | inhibition | 4.2±2 |
| 49 | fluoro | 2-hydroxy-5-chlorobenzylamino | inhibition | 10.8±3 |
| 50 | fluoro | 2-hydroxy-5-fluorobenzylamino | inhibition | 13.6±3 |

As P2Y₁-like and A2 purinergic receptors, negatively and positively coupled to adenylate cyclase respectively, are present on rat C6 glioma as to be determined if the modulation of the synthesis of cAMP is due to inhibition of the activation of β-adrenergic receptors by isoproterenol are due to activation of purinergic receptors.

The substituted 2-substituted-6-benzylaminopurine riboside derivatives demonstrated anti-inflammatory activity. Kinetin riboside was inactive in the test protocol.

### EXAMPLE 29

### Anti-inflammatory effects against neutrophils,

Anti-inflammatory effects against neutrophils were determined by assessing effects of several of the compounds on neutrophil inflammatory activity (superoxide generation, secretion of chemoattractant IL8 and degranulation to release myeloperoxidase). Neutrophils were isolated from human blood to a purity of greater than 95% and were cultured in RPMI1640 medium containing 2% human AB serum, 2 mM L-glutamine and antibiotics (100 U/ml penicillin, 100 mg/ml streptomycin). Neutrophils were primed for 30 minutes with 50 ng/ml GM-CSF prior to treatment with the bacterial stimulant fMLP (100 ng/ml) and measurement of superoxide generation in a lucigenin based assay (see Pongracz J and Lord JM 1998, *Biochem.Biophys.Res.Commun.* **247**, 624-629) and release of IL8 and myeloperoxidase using an ELISA method. The novel compounds were added to the culture at a range of concentrations (0.1 - 100 µM) 10 minutes prior to addition of fMLP. Compound 10 inhibited each of these aspects of the pro-inflammatory activity of neutrophils, with an IC₅₀ of 5 µM for superoxide generation (Fig. 2), 50 nM for IL8 release (Fig. 3) and 4 µM for degranulation and release of myeloperoxidase (Fig. 4).

Effects of the compounds on neutrophil apoptosis were also determined as an indication of utility in diseases where their survival is dysregulated such as systemic vasculitis and chronic inflammatory disease. Peripheral blood neutrophils were cultured for 20 hours in RPMI1640 medium containing 2% human AB serum, 2 mM L-glutamine and antibiotics (100 U/ml penicillin, 100 mg/ml streptomycin) and their apoptosis determined by staining with Giemsa stain and observing an apoptotic morphology by light microscopy (see Pongracz J et al. 1999, J.Biol.Chem. 274: 37329-37334). Compound 10 inhibited neurophil apoptosis with an IC₅₀ of 1 µM (Fig. 4).

The ability of neutrophils to adhere to vascular endothelium and migrate from the blood into tissue, is the first step in the inflammatory process. Interference with this process would also result in suppression of inflammation. Neutrophils were flowed over TNF primed endothelial cells (HUVEC) lining a capillary based migration system, at flows rates and pressures mimicking the in vivo vasculature (see for detailed description of apparatus). Cells were recorded using time-lapse video microscopy and the numbers of rolling, adhered and transmigrated cells were enumerated over a 20 minute period. In test incubations the neutrophils and HUVEC were pre-treated with compound 10 at 50 and 100 µM and the compound was also present in the medium in which neutrophils were suspended. The data show that compound 10 reduced the number of cells that adhered and transmigrated through the endothelial layer and increased the ones that rolled over the endothelium (Fig. 5).

### EXAMPLE 30

### Anti-neutrophil cytoplasmic antibody (ANCA) associated-vasculitis

ANCA are predominantly IgG autoantibodies directed against constituents of primary granules of neutrophils (PR3 and MPO). ANCA associated vasculitis is the most common primary systemic small-vessel vasculitis in adults. The common pathology of this disease is focal necrotizing lesions of blood vessels and may affect different vessels and organs. It is the most common cause of rapidly progressive glomerulonephritis-induced end-stage renal failure (ESRF). Previous studies have shown that neutrophil apoptosis is dysregulated and increased in ANCA patients, leading to their reduced uptake by macrophages and possible increased proinflammatory activity in systemic vessels. Therapies able to delay neutrophil apoptosis and reduce their inflammatory activity would be of utility in this condition. Neutrophils were cultured for 10 hours in RPMI1640 medium containing 2% human AB serum, 2 mM L-glutamine and antibiotics (100 U/ml penicillin, 100 mg/ml streptomycin) and their apoptosis determined by staining with Giemsa stain and observing an apoptotic morphology by light microscopy. Neutrophils were pre-treated for 10 minutes with 20 ng/ml TNFα to induce expression of ANCA epitopes on the surface of the neutrophils and ANCA (both anti-PR3 and anti-MPO) were included in the medium to increase apoptosis. Compound 10 was also able to overcome the increase in apoptosis imposed by the ANCA with an IC₅₀ of 4 µM (Figure 9). The ability of compound 10 to inhibit ANCA-induced neutrophil inflammatory functions was also determined. Neutrophils were cultured in RPMI1640 medium containing 2% human AB serum, 2 mM L-glutamine and antibiotics (100 U/ml penicillin, 100 mg/ml streptomycin). The medium also contained ANCA in the presence of absence of compound 10 and neutrophils were again primed with TNFα to induce responsiveness to ANCA. ANCA stimulated degranulation of neutrophils was assessed by release of myeloperoxidase in to the medium over a 30 minute period, with MPO measured by ELISA. Compound 10 inhibited neutrophil degranulation with an IC₅₀ of 3µM (Figure 10). In similar studies TNF primed-neutrophils were incubated with ANCA and a single concentration of compound 10 (10 µM) and superoxide generation was measured over a 10 minute period using a lucigenin based assay (see Pongracz J and Lord JM 1998, Biochem.Biophys.Res.Commun. 247, 624-629). This concentration of compound 10 completely inhibited ANCA-induced superoxide generation (Figure 11). A key pathology associated with systemic vasculitis is neutrophil mediated damage to endothelial cells. The determine whether compound 10 could reduce such damage, ANCA-activated TNF primed-neutrophils were incubated overnight with human umbilical vein endothelial cells (HUVEC) in the absence or presence of compound 10 (10 µM). HUVEC viability was determined by trypan blue staining and light microscopy. After 24h 50% cell death was seen with HUVEC treated with TNF-primed neutrophils and this increased to 90% in the presence of ANCA (Figure 12). Compound 10 was able to completely block the death of HUVEC treated with TNF-primed neutrophils and reduced cell death to 50% in the presence of ANCA.

### EXAMPLE 31: Dry Capsules

Five thousand capsules, each of which contains 0.25 g of one of the compounds of the formula I as active ingredient, are prepared as follows:

### Composition

| | |
|---|---|
| Active ingredient | 1250 g |
| Talc | 180 g |
| Wheat starch | 120 g |
| Magnesium stearate | 80 g |
| Lactose | 20 g |

Preparation process: The powdered substances mentioned are pressed through a sieve of mesh width 0.6 mm. Portions of 0.33 g of the mixture are transferred to gelatine capsules with the aid of a capsule-filling machine.

### EXAMPLE 32: Soft Capsules

Five thousand soft gelatine capsules, each of which contains 0.05 g of one of the compounds of the formula I as active ingredient, are prepared as follows:

### Composition

| | |
|---|---|
| Active ingredient | 250 g |
| Lauroglycol | 2 litres |

Preparation process: The powdered active ingredient is suspended in Lauroglykol^{®} (propylene glycol laurate, Gattefossé S.A., Saint Priest, France) and ground in a wet-pulveriser to a particle size of about 1 to 3 µm. Portions of in each case 0.419 g of the mixture are then transferred to soft gelatine capsules by means of a capsule-filling machine.

### EXAMPLE 33: Soft Capsules

Five thousand soft gelatine capsules, each of which contains 0.05 g of one of the compounds of the formula I as active ingredient, are prepared as follows:

### Composition

| | |
|---|---|
| Active ingredient | 250 g |
| PEG 400 | 1 litre |
| Tween 80 | 1 litre |

Preparation process: The powdered active ingredient is suspended in PEG 400 (polyethylene glycol of MW between 380 and about 420, Sigma, Fluka, Aldrich, USA) and Tween^{®} 80 (polyoxyethylene sorbitan monolaurate, Atlas Chem. Inc., Inc., USA, supplied by Sigma, Fluka, Aldrich, USA) and ground in a wet-pulveriser to a particle size of about 1 to 3 mm. Portions of in each case 0.43 g of the mixture are then transferred to soft gelatine capsules by means of a capsule-filling machine.

## Claims

1. 2-Substituted-6-(substituted benzylamino)purine riboside derivatives of the general formula I 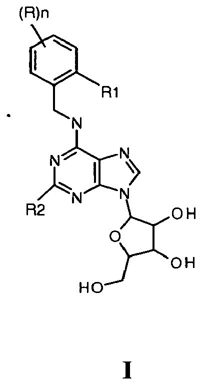 wherein
(**R**)ₙ represents 0 to 4 substituents **(n** is 0-4), which can be the same or different, the substituents being selected from the group comprising alkyl, alkoxy, amino, halogen, hydroxyl, nitro, mercapto and alkylmercapto, and
**R1** is selected from the group consisting of halogen, hydroxy, amino, alkoxy, mercapto, alkylmercapto, alkyl, and
R2 is selected from the group consisting of halogen, amino, azido, alkoxy, mercapto, alkylmercapto, alkyl, alkenyl, alkynyl, -NHNH₂, -NHOH, -NHCONH₂, guanylo (-NH-C(NH)NH₂), and
**R2** is **R2'-N(R3)-** wherein
**R2'** is selected from the group consisting of alkyl, alkenyl, cycloalkyl, phenyl and benzyl, wherein each of the groups can optionally be substituted by one or more substituents selected from the group comprising amino, halogen, hydroxy, alkoxy, mercapto and alkylmercapto,
**R3** is selected from hydrogen and alkyl, said alkyl being unsubstituted or substituted by one or more substituents selected from the group comprising amino, halogen, hydroxy, alkoxy, mercapto and alkylmercapto,
and the pharmaceutically acceptable salts thereof with alkali metals, ammonium or amines, or their addition salts with acids,
for use in therapeutical suppression of apoptosis and inflammatory activity of neutrophils.

2. The 2-substituted-6-(substituted benzylamino)purine riboside derivatives of the general formula **I** for use in therapeutical suppression of apoptosis and inflammatory activity of neutrophils according to claim 1, wherein the derivatives of the general formula **I** are selected from the group comprising 2-(amino, chloro, fluoro, mercapto, methyl, C₁-C₆-alkylmercapto, C₁-C₆-alkylamino, hydroxy(C₁-C₆)alkylamino, amino(C₁-C₆)alkylamino)-6-(2-hydroxy-3-methoxybenzylamino)purine riboside, 2-(amino, chloro, fluoro, mercapto, methyl, C₁-C₆-alkylmercapto, C₁-C₆-alkylamino, hydroxy(C₁-C₆)alkylamino, amino(C₁-C₆)alkylamino)-6-(2-hydroxy-4-methoxybenzylamino)purine riboside, 2-(amino, chloro, fluoro, mercapto, methyl, C₁-C₆-alkylmercapto, C₁-C₆-alkylamino, hydroxy(C₁-C₆)alkylamino, amino(C₁-C₆)alkylamino)-6-(2-hydroxy-5-methoxybenzylamino)-purine riboside, 2-(amino, chloro, fluoro , mercapto, methyl, C₁-C₆-alkylmercapto, C₁-C₆-alkylamino, hydroxy(C₁-C₆)alkylamino, amino(C₁-C₆)alkylamino)-6-(2-hydroxy-6-methoxybenzylamino)purine riboside, 2-(amino, chloro, fluoro , mercapto, C₁-C₆-alkylmercapto, C₁-C₆-alkylamino, hydroxy(C₁-C₆)alkylamino, amino(C₁-C₆)alkylamino)-6-(2,3-dihydroxy-4-methoxybenzylamino)purine riboside, 2-(amino, chloro, fluoro, , methyl, mercapto, C₁-C₆-alkylmercapto, C₁-C₆-alkylamino, hydroxy(C₁-C₆)alkylamino, amino(C₁-C₆)alkylamino)-6-(2,5-dihydroxy-4-methoxy-benzylamino)purine riboside, 2-(amino, chloro, fluoro , methyl, mercapto, C₁-C₆-alkylmercapto, C₁₋C₆-alkylamino, hydroxy(C₁-C₆)alkylamino, amino(C₁-C₆)alkylamino)-6-(2-hydroxy-3-methylbenzylamino)purine riboside, 2-(amino, chloro, fluoro, mercapto, methyl, C₁-C₆-alkylmercapto, C₁-C₆-alkylamino, hydroxy(C₁-C₆)alkylamino, amino(C₁-C₆)alkylamino)-6-(2-hydroxy-4-methylbenzylamino)purine riboside, 2-(amino, chloro, fluoro, mercapto, methyl, C₁-C₆-alkylmercapto, C₁-C₆-alkylamino, hydroxy(C₁-C₆)alkylamino, amino(C₁-C₆)alkylamino)-6-(2-hydroxy-5-methylbenzylamino)purine riboside, 2-(amino, chloro, fluoro, mercapto, methyl, C₁-C₆-alkylmercapto, C₁-C₆-alkylamino, hydroxy(C₁-C₆)alkylamino, amino(C₁-C₆)alkylamino)-6-(2-hydroxy-6-methylbenzylamino)purine riboside, 2-(amino, chloro, fluoro, mercapto, methyl, C₁-C₆-alkylmercapto, C₁-C₆-alkylamino, hydroxy(C₁-C₆)alkylamino, amino(C₁-C₆)alkylamino)-6-(2-hydroxy-3-chlorobenzylamino)purine riboside, 2-(amino, chloro, fluoro, mercapto, methyl, C₁-C₆-alkylmercapto, C₁-C₆-alkylamino, hydroxy(C₁-C₆)alkylamino, amino(C₁-C₆)alkylamino)-6-(2-hydroxy-4-chlorobenzylamino)purine riboside, 2-(amino, chloro, fluoro, mercapto, methyl, C₁-C₆-alkylmercapto, C₁-C₆-alkylamino, hydroxy(C₁-C₆)alkylamino, amino(C₁-C₆)alkylamino)-6-(2-hydroxy-5-chlorobenzylamino)purine riboside, 2-(amino, chloro, fluoro, mercapto, methyl, C₁-C₆-alkylmercapto, C₁-C₆-alkylamino, hydroxy(C₁-C₆)alkylamino, amino(C₁-C₆)alkylamino)-6-(2-hydroxy-6-chlorobenzylamino)purine riboside, 2-(amino, chloro, fluoro, mercapto, methyl, C₁-C₆-alkylmercapto, C₁-C₆-alkylamino, hydroxy(C₁-C₆)alkylamino, amino(C₁-C₆)alkylamino)-6-(2-hydroxy-3-fluorobenzylamino)purine riboside, 2-(amino, chloro, fluoro , mercapto, methyl, C₁-C₆-alkylmercapto, C₁-C₆-alkylamino, hydroxy(C₁-C₆)alkylamino, amino(C₁-C₆)alkylamino)-6-(2-hydroxy-5-fluorobenzylamino)purine riboside, 2-(amino, chloro, fluoro, mercapto, methyl, C₁-C₆-alkylmercapto, C₁-C₆-alkylamino, hydroxy(C₁-C₆)alkylamino, amino(C₁-C₆)alkylamino)-6-(2-hydroxy-3-bromobenzylamino)purine riboside, 2-(amino, chloro, fluoro, mercapto, methyl, C₁-C₆-alkylmercapto, C₁-C₆-alkylamino, hydroxy(C₁-C₆)alkylamino, amino(C₁-C₆)alkylamino)-6-(2-hydroxy-3-iodobenzylamino)purine riboside, 2-(amino, chloro, fluoro, mercapto, methyl, C₁-C₆-alkylmercapto, C₁-C₆-alkylamino, hydroxy(C₁-C₆)alkylamino, amino(C₁-C₆)alkylamino)-6-(2,3-dihydroxy-4-methylbenzylamino) purine riboside, 2-(amino, chloro, fluoro, mercapto, methyl, C₁-C₆-alkylmercapto, C₁-C₆-alkylamino, hydroxy(C₁-C₆)alkylamino, amino(C₁-C₆)alkylamino)-6-(2,5-dihydroxy-4-methylbenzylamino)purine riboside, 2-(amino, chloro, fluoro, mercapto, methyl, C₁-C₆-alkylmercapto, C₁-C₆-alkylamino, hydroxy(C₁-C₆)alkylamino, amino(C₁-C₆)alkylamino)-6-(2,3-dihydroxy-4-chlorobenzylamino)purine riboside, 2-(amino, chloro, fluoro, mercapto, methyl, C₁-C₆-alkylmercapto, C₁-C₆-alkylamino, hydroxy(C₁-C₆)alkylamino, amino(C₁-C₆)alkylamino)-6-(2,5-dihydroxy-4-chlorobenzylamino)purine riboside, 2-(amino, chloro, fluoro, mercapto, methyl, C₁-C₆-alkylmercapto, C₁-C₆-alkylamino, hydroxy(C₁-C₆)alkylamino, amino(C₁-C₆)alkylamino)-6-(2,6-dihydroxy-4-chlorobenzylamino) purine riboside, 2-(amino, chloro, fluoro, mercapto, methyl, C₁-C₆-alkylmercapto, C₁-C₆-alkylamino, hydroxy(C₁-C₆)alkylamino, amino(C₁-C₆)alkylamino)-6-(2-hydroxy-3-aminobenzylamino)purine riboside, 2-(amino, chloro, fluoro, mercapto, methyl, C₁-C₆-alkylmercapto, C₁-C₆-alkylamino, hydroxy(C₁-C₆)alkylamino, amino(C₁-C₆)alkylamino)-6-(2-hydroxy-4-aminobenzylamino)purine riboside, 2-(amino, chloro, fluoro, methyl, mercapto, C₁-C₆-alkylmercapto, C₁-C₆-alkylamino, hydroxy(C₁-C₆)alkylamino, amino(C ₁-C₆)alkylamino)-6-(2-hydroxy-5-aminobenzylamino)purine riboside, 2-(amino, chloro, methyl,, methyl, mercapto, C₁-C₆-alkylmercapto, C₁-C₆-alkylamino, hydroxy(C₁-C₆)alkylamino, amino(C₁-C₆)alkylamino)-6-(2-hydroxy-5-mercaptobenzylamino)purine riboside, 2-(amino, chloro, fluoro, methyl, mercapto, C₁-C₆-alkylmercapto, C₁-C₆-alkylamino, hydroxy(C₁-C₆)alkylamino, amino(C₁-C₆)alkylamino)-6-(2-amino-3-methoxybenzylamino)purine riboside, 2-(amino, chloro, fluoro, methyl,mercapto, C₁-C₆-alkylmercapto, C₁-C₆-alkylamino, hydroxy(C₁-C₆)alkylamino, amino(C₁-C₆)alkylamino)-6-(2-amino-4-methoxybenzylamino)purine riboside, 2-(amino, chloro, fluoro, methyl,mercapto, C₁-C₆-alkylmercapto, C₁-C₆-alkylamino, hydroxy(C₁-C₆)alkylamino, amino(C₁-C₆)alkylamino)-6-(2-amino-5-methoxybenzylamino)purine riboside, 2-(amino, chloro, fluoro, methyl, mercapto, C₁-C₆-alkylmercapto, C₁-C₆-alkylamino, hydroxy(C₁-C₆)alkylamino, amino(C₁-C₆)alkylamino)-6-(2-amino-3-methylbenzylamino)purine riboside, 2-(amino, chloro, fluoro, methyl,, mercapto, C₁-C₆-alkylmercapto, C₁-C₆-alkylamino, hydroxy(C₁-C₆)alkylamino, amino(C₁-C₆)alkylamino)-6-(2-amino-5-methylbenzylamino)purine riboside, 2-(amino, chloro, fluoro, methyl, mercapto, C₁-C₆-alkylmercapto)-6-(2-amino-3-chlorobenzylamino)purine riboside, 2-(amino, chloro, fluoro, methyl, mercapto, C₁-C₆-alkylmercapto, C₁-C₆-alkylamino, hydroxy(C₁-C₆)alkylamino, amino(C₁-C₆)alkylamino)-6-(2-amino-4-chlorobenzylamino)purine riboside, 2-(amino, chloro, fluoro, methyl, mercapto, C₁-C₆-alkylmercapto, C₁-C₆-alkylamino, hydroxy(C ₁-C₆)alkylamino, amino(C₁-C₆)alkylamino)-6-(2-amino-5-chlorobenzylamino)purine riboside, 2-(amino, chloro, fluoro, methyl, mercapto, C₁-C₆-alkylmercapto, C₁-C₆-alkylamino, hydroxy(C₁-C₆)alkylamino, amino(C₁-C₆)alkylamino)-6-(2-amino-6-chlorobenzylamino)purine riboside, 2-(amino, chloro, fluoro, methyl, mercapto, C₁-C₆-alkylmercapto, C₁-C₆-alkylamino, hydroxy(C₁-C₆)alkylamino, amino(C₁-C₆)alkylamino)-6-(2-amino-3-fluorobenzylamino)purine riboside, 2-(amino, chloro, fluoro, methyl,, mercapto, C₁-C₆-alkylmercapto, C₁-C₆-alkylamino, hydroxy(C₁-C₆)alkylamino, amino(C₁-C₆)alkylamino)-6-(2-amino-5-fluorobenzylamino)purine riboside, 2-(amino, chloro, fluoro, methyl, mercapto, C₁-C₆-alkylmercapto, C₁-C₆-alkylamino, hydroxy(C₁-C₆)alkylamino, amino(C₁-C₆)alkylamino)-6-(2-amino-3-bromobenzylamino)purine riboside, 2-(amino, chloro, fluoro, methyl,, mercapto, C₁-C₆-alkylmercapto, C₁-C₆-alkylamino, hydroxy(C₁-C₆)alkylamino, amino(C₁-C₆)alkylamino)-6-(2,3-diaminobenzylamino)purine riboside, 2-(amino, chloro, fluoro, methyl, mercapto, C₁-C₆-alkylmercapto, C₁-C₆-alkylamino, hydroxy(C₁-C₆)alkylamino, amino(C₁-C₆)alkylamino)-6-(2,5-diaminobenzylamino)purine riboside, 2-(amino, chloro, fluoro, methyl,, mercapto, C₁-C₆-alkylmercapto, C₁-C₆-alkylamino, hydroxy(C₁-C₆)alkylamino, amino(C₁-C₆)alkylamino)-6-(2,6-diamino-3-chlorobenzylamino)purine riboside, 2-(amino, chloro, fluoro, methyl, mercapto, C₁-C₆-alkylmercapto, C₁-C₆-alkylamino, hydroxy(C₁-C₆)alkylamino, amino(C₁-C₆)alkylamino)-6-(2,6-diamino-4-chlorobenzylamino)purine riboside, 2-(amino, chloro, fluoro, methyl, mercapto, C₁-C₆-alkylmercapto, C₁-C₆-alkylamino, hydroxy(C₁-C₆)alkylamino, amino(C₁-C₆)alkylamino)-6-(2,3-diamino-4-chlorobenzylamino)purine riboside,
and the pharmaceutically acceptable salts thereof with alkali metals, ammonium or amines, or their addition salts with acids.

3. The 2-substituted-6-(substituted benzylamino)purine riboside derivatives of the general formula I for use in therapeutical suppression of apoptosis and inflammatory activity of neutrophils according to claim 1, wherein the derivatives of the general formula I are selected from the group comprising 2-(chloro, fluoro, C₁-C₆-alkylmercapto, C₁-C₆-alkylamino, hydroxy(C₁-C₆)alkylamino, amino(C₁-C₆)alkylamino)-6-(2-hydroxy-3-methoxybenzylamino)-purine riboside, 2-(chloro, fluoro, C₁-C₆-alkylmercapto, C₁-C₆-alkylamino, hydroxy(C₁-C₆)alkylamino, amino(C₁-C₆)alkylamino)-6-(2-hydroxy-5-methoxybenzylamino)purine riboside, 2-(chloro, fluoro, C₁-C₆-alkylmercapto, C₁-C₆-alkylamino, hydroxy(C₁-C₆)alkylamino, amino(C₁-C₆)alkylamino)-6-(2-hydroxy-3-methylbenzylamino)purine riboside, 2-(chloro, fluoro, C₁-C₆-alkylmercapto; C₁-C₆-alkylamino, hydroxy(C₁-C₆)alkylamino, amino(C₁-C₆)alkylamino)-6-(2-hydroxy-5-methylbenzylamino)purine riboside, 2-(chloro, fluoro, C₁-C₆-alkylmercapto, C₁-C₆-alkylamino, hydroxy(C₁-C₆)alkylamino, amino(C₁-C₆)alkylamino)-6-(2-hydroxy-3-chlorobenzylamino)purine riboside, 2-(chloro, fluoro, C₁-C₆-alkylmercapto, C₁-C₆-alkylamino, hydroxy(C₁-C₆)alkylamino, amino(C₁-C₆)alkylamino)-6-(2-hydroxy-5-chlorobenzylamino)purine riboside, 2-(chloro, fluoro, C₁-C₆-alkylmercapto, C₁-C₆-alkylamino, hydroxy(C₁-C₆)alkylamino, amino(C₁-C₆)alkylamino)-6-(2-hydroxy-5-fluorobenzyl-amino)purine riboside, 2-(chloro, fluoro, C₁-C₆-alkylmercapto, C₁-C₆-alkylamino, hydroxy(C₁-C₆)alkylamino, amino(C₁-C₆)alkylamino)-6-(2-amino-5-chlorobenzylamino)purine riboside, 2-(chloro, fluoro, C₁-C₆-alkylmercapto, C₁-C₆-alkylamino, hydroxy(C₁-C₆)alkylamino, amino(C₁-C₆)alkylamino)-6-(2-amino-5-fluorobenzylamino)purine riboside 2-(chloro, fluoro, C₁-C₆-alkylmercapto, C₁-C₆-alkylamino, hydroxy(C₁-C₆)alkylamino, amino(C₁-C₆)alkylamino)-6-(2-hydroxy-5-aminobenzylamino)purine riboside, and the pharmaceutically acceptable salts thereof with alkali metals, ammonium or amines, or their addition salts with acids.

4. The 2-substituted-6-(substituted benzylamino)purine riboside derivatives of the general formula I according to any of claims 1 to 3 for use in the treatment or prophylaxis of inflammatory diseases.

5. The 2-substituted-6-(substituted benzylamino)purine riboside derivatives of the general formula I according to any of claims 1 to 3 for use in the treatment of a disease selected from the group comprising ANCA-associated vasculitis, glomerulonephritis-induced progressive renal failure (ESRF), neutropenia, emphysema, familial Mediterranean fever (FMF), arthralgia, peritonitis, amyloidosis, chronical inflammatory diseases.

6. Use of the 2-substituted-6-(substituted benzylamino)purine riboside derivatives of the general formula I according to any of claims 1 to 3 as anti-apoptotic factors of plant and animal cells in tissue cultures.

## Patentansprüche

1. 2-Substituierte-6-substituierte-benzylaminopurinriboside der allgemeinen Formel I 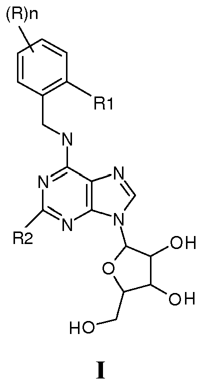 worin
**(R)**ₙ 0 bis 4 Substituenten (n entspricht 0 bis 4) bedeutet, welche identisch oder unterschiedlich sein können, wobei diese Substituenten aus einer Gruppe umfassend Alkyl, Alkoxy, Amino, Halogen, Hydroxyl, Nitro, Merkapto und Alkylmerkapto ausgewählt sind, und
worin **R1** aus einer Gruppe umfassend Halogen, Hydroxy, Amino, Alkoxy, Merkapto, Alkylmerkapto, Alkyl ausgewählt ist, und
worin R2 aus einer Gruppe umfassend Halogen, Amino, Azido, Alkoxy, Merkapto, Alkylmerkapto, Alkyl, Alkenyl, Alkynyl, -NHNH₂, -NHOH, -NHCONH₂, Guanylo (-NH-C(NH)NH₂) ausgewählt ist, und
worin R2 R2'-N(R3)- bedeutet, worin
R2' aus einer Gruppe umfassend Alkyl, Alkenyl, Cykloalkyl, Phenyl und Benzyl ausgewählt ist, wobei jede Gruppe allfällig durch einen oder mehrere Substituenten substituiert werden kann, ausgewählt aus einer Gruppe umfassend Amino, Halogen, Hydroxy, Alkoxy, Merkapto und Alkylmerkapto,
R3 aus Wasserstoff und Alkyl ausgewählt ist, wobei dieses Alkyl nicht substituiert ist oder durch einen oder mehrere Substituenten substituiert ist, ausgewählt aus einer Gruppe umfassend Amino, Halogen, Hydroxy, Alkoxy, Merkapto und Alkylmerkapto,
und deren pharmazeutisch akzeptable Salze mit Alkalimetallen, Ammoniak oder Aminen, oder deren Additionssalze mit Säuren,
zur Verwendung bei der therapeutischen Suppression der Apoptose und der entzündlichen Wirkung von Neutrophilen.

2. 2-Substituierte-6-substituierte-benzylaminopurinriboside der allgemeinen Formel I zur Verwendung bei der therapeutischen Suppression der Apoptose und der entzündlichen Wirkung von Neutrophilen nach dem Anspruch 1, wobei die Derivate der allgemeinen Formel I ausgewählt sind aus einer Gruppe umfassend 2-(Amino, Chlor, Fluor, Merkapto, Methyl, C₁-C₆-Alkylmerkapto, C₁-C₆-Alkylamino, Hydroxy(C₁-C₆)alkylamino, Amino(C₁-C₆)alkylamino)-6-(2-hydroxy-3-methoxybenzylamino)purin-ribosid, 2-(Amino, Chlor, Fluor, Merkapto, Methyl, C₁-C₆-Alkylmerkapto, C₁-C₆-Alkylamino, Hydroxy(C₁-C₆)alkylamino, Amino(C₁-C₆)alkylamino)-6-(2-hydroxy-4-methoxybenzylamino)purinribosid, 2-(Amino, Chlor, Fluor, Merkapto, Methyl, C₁-C₆-Alkylmerkapto, C₁-C₆-Alkylamino, Hydroxy(C₁-C₆)alkylamino, Amino(C₁-C₆)alkylamino)-6-(2-hydroxy-5-methoxybenzylamino) purinribosid, 2-(Amino, Chlor, Fluor, Merkapto, Methyl, C₁-C₆-Alkylmerkapto, C₁-C₆-Alkylamino, Hydroxy(C₁-C₆)alkylamino, Amino(C₁-C₆)alkylamino)-6-(2-hydroxy-6-methoxybenzylamino)purinribosid, 2-(Amino, Chlor, Fluor, Merkapto, C₁-C₆-Alkylmerkapto, C₁-C₆-Alkylamino, Hydroxy(C₁-C₆)alkylamino, Amino(C₁-C₆)alkylamino)-6-(2,3-dihydroxy-4-methoxybenzylamino)purinribosid, 2-(Amino, Chlor, Fluor, Methyl, Merkapto, C₁-C₆-Alkylmerkapto, C₁-C₆-Alkylamino, Hydroxy(C₁-C₆)alkylamino, Amino(C₁-C₆)alkylamino)-6-(2,5-dihydroxy-4-methoxybenzylamino) purinribosid, 2-(Amino, Chlor, Fluor, Methyl, Merkapto, C₁-C₆-Alkylmerkapto, C₁-C₆-Alkylamino, Hydroxy(C₁-C₆)alkylamino, Amino(C₁-C₆)alkylamino)-6-(2-hydroxy-3-methylbenzylamino)purinribosid, 2-(Amino, Chlor, Fluor, Merkapto, Methyl, C₁-C₆-Alkylmerkapto, C₁-C₆-Alkylamino, Hydroxy(C₁-C₆)alkylamino, Amino(C₁-C₆)alkylamino)-6-(2-hydroxy-4-methylbenzylamino)purinribosid, 2-(Amino, Chlor, Fluor, Merkapto, Methyl, C₁-C₆-Alkylmerkapto, C₁-C₆-Alkylamino, Hydroxy(C₁-C₆)alkylamino, Amino(C₁-C₆)alkylamino)-6-(2-hydroxy-5-methylbenzylamino) purinribosid, 2-(Amino, Chlor, Fluor, Merkapto, Methyl, C₁-C₆-Alkylmerkapto, C₁-C₆-Alkylamino, Hydroxy(C₁-C₆)alkylamino, Amino(C₁-C₆)alkylamino)-6-(2-hydroxy-6-methylbenzylamino)purinribosid, 2-(Amino, Chlor, Fluor, Merkapto, Methyl, C₁-C₆-Alkylmerkapto, C₁-C₆-Alkylamino, Hydroxy(C₁-C₆)alkylamino, Amino(C₁-C₆)alkylamino)-6-(2-hydroxy-3-chlorbenzylamino)purinribosid, 2-(Amino, Chlor, Fluor, Merkapto, Methyl, C₁-C₆-Alkylmerkapto, C₁-C₆-Alkylamino, Hydroxy(C₁-C₆)alkylamino, Amino(C₁-C₆)alkylamino)-6-(2-hydroxy-4-chlorbenzylamino)purinribosid, 2-(Amino, Chlor, Fluor, Merkapto, Methyl, C₁-C₆-Alkylmerkapto, C₁-C₆-Alkylamino, Hydroxy(C₁-C₆)alkylamino, Amino(C₁-C₆)alkylamino)-6-(2-hydroxy-5-chlorbenzylamino)purinribosid, 2-(Amino, Chlor, Fluor, Merkapto, Methyl, C₁-C₆-Alkylmerkapto, C₁-C₆-Alkylamino, Hydroxy(C₁-C₆)alkylamino, Amino(C₁-C₆)alkylamino)-6-(2-hydroxy-6-chlorbenzylamino)purinribosid, 2-(Amino, Chlor, Fluor, Merkapto, Methyl, C₁-C₆-Alkylmerkapto, C₁-C₆-Alkylamino, Hydroxy(C₁-C₆)alkylamino, Amino(C₁-C₆)alkylamino)-6-(2-hydroxy-3-fluorbenzylamino) purinribosid, 2-(Amino, Chlor, Fluor, Merkapto, Methyl, C₁-C₆-Alkylmerkapto, C₁-C₆-Alkylamino, Hydroxy(C₁-C₆)alkylamino, Amino(C₁-C₆)alkylamino)-6-(2-hydroxy-5-fluorbenzylamino)purinribosid, 2-(Amino, Chlor, Fluor, Merkapto, Methyl, C₁-C₆-Alkylmerkapto, C₁-C₆-Alkylamino, Hydroxy(C₁-C₆)alkylamino, Amino(C₁-C₆)alkylamino)-6-(2-hydroxy-3-brombenzylamino)purinribosid, 2-(Amino, Chlor, Fluor, Merkapto, Methyl, C₁-C₆-Alkylmerkapto, C₁-C₆-Alkylamino, Hydroxy(C₁-C₆)alkylamino, Amino(C₁-C₆)alkylamino)-6-(2-hydroxy-3-jodobenzylamino) purinribosid, 2-(Amino, Chlor, Fluor, Merkapto, Methyl, C₁-C₆-Alkylmerkapto, C₁-C₆-Alkylamino, Hydroxy(C₁-C₆)alkylamino, Amino(C₁-C₆)alkylamino)-6-(2,3-dihydroxy-4-methylbenzylamino)purinribosid, 2-(Amino, Chlor, Fluor, Merkapto, Methyl, C₁-C₆-Alkylmerkapto, C₁-C₆-Alkylamino, Hydroxy(C₁-C₆)alkylamino, Amino(C₁-C₆)alkylamino)-6-(2,5-dihydroxy-4-methylbenzylamino)purinribosid, 2-(Amino, Chlor, Fluor, Merkapto, Methyl, C₁-C₆-Alkylmerkapto, C₁-C₆-Alkylamino, Hydroxy(C₁-C₆)alkylamino, Amino(C₁-C₆)alkylamino)-6-(2,3-dihydroxy-4-chlorbenzylamino)purinribosid, 2-(Amino, Chlor, Fluor, Merkapto, Methyl, C₁-C₆-Alkylmerkapto, C₁-C₆-Alkylamino, Hydroxy(C₁-C₆)alkylamino, Amino(C₁-C₆)alkylamino)-6-(2,5-dihydroxy-4-chlorbenzylamino)purinribosid, 2-(Amino, Chlor, Fluor, Merkapto, Methyl, C₁-C₆-Alkylmerkapto, C₁-C₆-Alkylamino, Hydroxy(C₁-C₆)alkylamino, Amino(C₁-C₆)alkylamino)-6-(2,6-dihydroxy-4-chlorbenzylamino)purinribosid, 2-(Amino, Chlor, Fluor, Merkapto, Methyl, C₁-C₆-Alkylmerkapto, C₁-C₆-Alkylamino, Hydroxy(C₁-C₆)alkylamino, Amino(C₁-C₆)alkylamino)-6-(2-hydroxy-3-aminobenzylamino) purinribosid, 2-(Amino, Chlor, Fluor, Merkapto, Methyl, C₁-C₆-Alkylmerkapto, C₁-C₆-Alkylamino, Hydroxy(C₁-C₆)alkylamino, Amino(C₁-C₆)alkylamino)-6-(2-hydroxy-4-aminobenzylamino)purinribosid, 2-(Amino, Chlor, Fluor, Merkapto, Methyl, C₁-C₆-Alkylmerkapto, C₁-C₆-Alkylamino, Hydroxy(C₁-C₆)alkylamino, Amino(C₁-C₆)alkylamino)-6-(2-hydroxy-5-aminobenzylamino)purinribosid, 2-(Amino, Chlor, Methyl, Merkapto, C₁-C₆-Alkylmerkapto, C₁-C₆-Alkylamino, Hydroxy(C₁-C₆)alkylamino, Amino(C₁-C₆)alkylamino)-6-(2-hydroxy-5-merkaptobenzylamino) purinribosid, 2-(Amino, Chlor, Fluor, Merkapto, Methyl, C₁-C₆-Alkylmerkapto, C₁-C₆-Alkylamino, Hydroxy(C₁-C₆)alkylamino, Amino(C1₋C6₎alkylamino)-6-(2-amino-3-methoxybenzylamino)purinribosid, 2-(Amino, Chlor, Fluor, Methyl, Merkapto, C₁-C₆-Alkylmerkapto, C₁-C₆-Alkylamino, Hydroxy(C₁-C₆)alkylamino, Amino(C₁-C₆)alkylamino)-6-(2-amino-4-methoxybenzylamino)purinribosid, 2-(Amino, Chlor, Fluor, Methyl, Merkapto, C₁-C₆-Alkylmerkapto, C₁-C₆-Alkylamino, Hydroxy(C₁-C₆)alkylamino, Amino(C₁-C₆)alkylamino)-6-(2-amino-5-methoxybenzylamino)purinribosid, 2-(Amino, Chlor, Fluor, Methyl, Merkapto, C₁-C₆-Alkylmerkapto, C₁-C₆-Alkylamino, Hydroxy(C₁-C₆)alkylamino, Amino(C₁-C₆)alkylamino)-6-(2-amino-3-methylbenzylamino)purinribosid, 2-(Amino, Chlor, Fluor, Methyl, Merkapto, C₁-C₆-Alkylmerkapto, C₁-C₆-Alkylamino, Hydroxy(C₁-C₆)alkylamino, Amino(C₁-C₆)alkylamino)-6-(2-amino-5-methylbenzylamino)purinriboside, 2-(Amino, Chlor, Fluor, Methyl, Merkapto, C₁-C₆-Alkylmerkapto)-6-(2-amino-3-chlorbenzylamino)purinribosid, 2-(Amino, Chlor, Fluor, Methyl, Merkapto, C₁-C₆-Alkylmerkapto, C₁-C₆-Alkylamino, Hydroxy(C₁-C₆)alkylamino, Amino(C₁-C₆)alkylamino)-6-(2-amino-4-chlorbenzylamino)purinribosid, 2-(Amino, Chlor, Fluor, Methyl, Merkapto, C₁-C₆-Alkylmerkapto, C₁-C₆-Alkylamino, Hydroxy(C₁-C₆)alkylamino, Amino(C₁-C₆)alkylamino)-6-(2-amino-5-chlorbenzylamino)purinribosid, 2-(Amino, Chlor, Fluor, Methyl, Merkapto, C₁-C₆-Alkylmerkapto, C₁-C₆-Alkylamino, Hydroxy(C₁-C₆)alkylamino, Amino(C₁-C₆)alkylamino)-6-(2-amino-6-chlorbenzylamino)purinribosid, 2-(Amino, Chlor, Fluor, Methyl, Merkapto, C₁-C₆-Alkylmerkapto, C₁-C₆-Alkylamino, Hydroxy(C₁-C₆)alkylamino, Amino(C₁-C₆)alkylamino)-6-(2-amino-3-fluorbenzylamino) purinribosid, 2-(Amino, Chlor, Fluor, Methyl, Merkapto, C₁-C₆-Alkylmerkapto, C₁-C₆-Alkylamino, Hydroxy(C₁-C₆)alkylamino, Amino(C₁-C₆)alkylamino)-6-(2-amino-5-fluorbenzylamino)purinribosid, 2-(Amino, Chlor, Fluor, Methyl, Merkapto, C₁-C₆-Alkylmerkapto, C₁-C₆-Alkylamino, Hydroxy(C₁-C₆)alkylamino, Amino(C₁-C₆)alkylamino)-6-(2-amino-3-brombenzylamino)purinribosid, 2-(Amino, Chlor, Fluor, Methyl, Merkapto, C₁-C₆-Alkylmerkapto, C₁-C₆-Alkylamino, Hydroxy(C₁-C₆)alkylamino, Amino(C₁-C₆)alkylamino)-6-(2,3-diaminobenzylamino)purinribosid, 2-(Amino, Chlor, Fluor, Methyl, Merkapto, C₁-C₆-Alkylmerkapto, C₁-C₆-Alkylamino, Hydroxy(C₁-C₆)alkylamino, Amino(C₁-C₆)alkylamino)-6-(2,5-diaminobenzylamino) purinribosid, 2-(Amino, Chlor, Fluor, Methyl, Merkapto, C₁-C₆-Alkylmerkapto, C₁-C₆-Alkylamino, Hydroxy(C₁-C₆)alkylamino, Amino(C₁-C₆)alkylamino)-6-(2,6-diamino-3-chlorbenzylamino)purinribosid, 2-(Amino, Chlor, Fluor, Methyl, Merkapto, C₁-C₆-Alkylmerkapto, C₁-C₆-Alkylamino, Hydroxy(C₁-C₆)alkylamino, Amino(C₁-C₆)alkylamino)-6-(2,6-diamino-4-chlorbenzylamino)purinribosid, 2-(Amino, Chlor, Fluor, Methyl, Merkapto, C₁-C₆-Alkylmerkapto, C₁-C₆-Alkylamino, Hydroxy(C₁-C₆)alkylamino, Amino(C₁-C₆)alkylamino)-6-(2,3-diamino-4-chlorbenzylamino) purinribosid,
und deren pharmazeutisch akzeptable Salze mit Alkalimetallen, Ammoniak oder Aminen, oder deren Additionssalze mit Säuren.

3. 2-Substituierte-6-substituierte-benzylaminopurinriboside der allgemeinen Formel I zur Verwendung bei der therapeutischen Suppression der Apoptose und der entzündlichen Wirkung von Neutrophilen nach dem Anspruch 1, wobei die Derivate der allgemeinen Formel I ausgewählt sind aus einer Gruppe umfassend 2-(Chlor, Fluor, C₁-C₆-Alkylmerkapto, C₁-C₆-Alkylamino, Hydroxy(C₁-C₆)alkylamino, Amino(C₁-C₆)alkylamino)-6-(2-hydroxy-3-methoxybenzylamino)purinribosid, 2-(Chlor, Fluor, C₁-C₆-Alkylmerkapto, C₁-C₆-Alkylamino, Hydroxy(C₁-C₆)alkylamino, Amino(C₁-C₆)alkylamino)-6-(2-hydroxy-5-methoxybenzylamino)purinribosid, 2-(Chlor, Fluor, C₁-C₆-Alkylmerkapto, C₁-C₆-Alkylamino, Hydroxy(C₁-C₆)alkylamino, Amino(C₁-C₆)alkylamino)-6-(2-hydroxy-3-methylbenzylamino)purinribosid, 2-(Chlor, Fluor, C₁-C₆-Alkylmerkapto, C₁-C₆-Alkylamino, Hydroxy(C₁-C₆)alkylamino, Amino(C₁-C₆)alkylamino)-6-(2-hydroxy-5-methylbenzylamino)purinribosid, 2-(Chlor, Fluor, C₁-C₆-Alkylmerkapto, C₁-C₆-Alkylamino, Hydroxy(C₁-C₆)alkylamino, Amino(C₁-C₆)alkylamino)-6-(2-hydroxy-3-chlorbenzylamino)purinribosid, 2-(Chlor, Fluor, C₁-C₆-Alkylmerkapto, C₁-C₆-Alkylamino, Hydroxy(C₁-C₆)alkylamino, Amino(C₁-C₆)alkylamino)-6-(2-hydroxy-5-chlorbenzylamino)purinribosid, 2-(Chlor, Fluor, C₁-C₆-Alkylmerkapto, C₁-C₆-Alkylamino, Hydroxy(C₁-C₆)alkylamino, Amino(C₁-C₆)alkylamino)-6-(2-hydroxy-5-fluorbenzylamino)purinribosid, 2-(Chlor, Fluor, C₁-C₆-Alkylmerkapto, C₁-C₆-Alkylamino, Hydroxy(C₁-C₆)alkylamino, Amino(C₁-C₆)alkylamino)-6-(2-amino-5-chlorbenzylamino)purinribosid, 2-(Chlor, Fluor, C₁-C₆-Alkylmerkapto, C₁-C₆-Alkylamino, Hydroxy(C₁-C₆)alkylamino, Amino(C₁-C₆)alkylamino)-6-(2-amino-5-fluorbenzylamino)purinribosid, 2-(Chlor, Fluor, C₁-C₆-Alkylmerkapto, C₁-C₆-Alkylamino, Hydroxy(C₁-C₆)alkylamino, Amino(C₁-C₆)alkylamino)-6-(2-hydroxy-5-aminobenzylamino)purinribosid,
und deren pharmazeutisch akzeptable Salze mit Alkalimetallen, Ammoniak oder Aminen, oder deren Additionssalze mit Säuren.

4. 2-Substituierte-6-substituierte-benzylaminopurinriboside der allgemeinen Formel I nach einem der Ansprüche 1 bis 3 zur Verwendung bei der Behandlung oder Prophylaxe entzündlicher Erkrankungen.

5. 2-Substituierte-6-substituierte-benzylaminopurinriboside der allgemeinen Formel I nach einem der Ansprüche 1 bis 3 zur Verwendung bei der Behandlung von Erkrankungen, ausgewählt aus einer Gruppe umfassend die ANCA-assoziierte Vasculitide, die Glomerulonephritis-induzierte progressive terminale Niereninsuffizienz (ESFR), Neutropenia, Emphysema, das Familiäre Mittelmeerfieber (FMF), Arthralgie, Peritonitis, Amyloidose, chronische entzündliche Erkrankungen.

6. Die Verwendung der 2-Substituierten-6-substituierten-benzylaminopurinriboside der allgemeinen Formel I nach einem der Ansprüche 1 bis 3 als antiapoptotische Faktoren der pflanzlichen und tierischen Zellen in den Gewebekulturen.

## Revendications

1. Dérivés de riboside de 6-(benzylamino substitué) purine 2-substituée de formule générale I 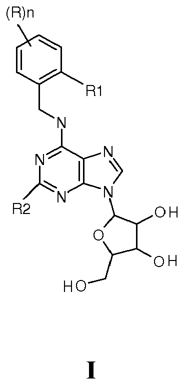
dans laquelle **(R)**ₙ représente 0 à 4 substituants (n est de 0 à 4), lesquels peuvent être égaux ou différents, ces substituants étant choisis parmi un groupe comprenant l'alkyle, alkoxy, amino, halogène, hydroxyle, nitro, mercapto et alkylmercapto, et
**R1** est choisi parmi un groupe comprenant le halogène, hydroxy, amino, alkoxy, mercapto, alkylmercapto, alkyle, et
R2 est choisi parmi un groupe comprenant le halogène, amino, azido, alkoxy, mercapto, alkylmercapto, alkyle, alcényle, alcynyle, -NHNH₂, -NHOH, -NHCONH₂, guanylo (-NH-C(NH)NH₂), et
R2 est R2'-N(R3)- dans lequel
R2' est choisi parmi un groupe comprenant l'alkyle, alcényle, cycloalkyle, phényle et benzyle, chaque groupe pouvant être éventuellement substitué par un ou plusieurs substituants choisis parmi un groupe comprenant l'amino, halogène, hydroxy, alkoxy, mercapto et alkylmercapto,
R3 est choisi dans l'hydrogène et l'alkyle, l'alkyle pouvant être non substitué ou substitué par un ou plusieurs substituants choisis parmi un groupe comprenant l'amino, halogène, hydroxy, alkoxy, mercapto et alkylmercapto,
et leurs sels pharmaceutiquement acceptables avec les métaux alcalins, l'ammoniaque ou les amines, ou leurs sels d'addition avec les acides, pour l'utilisation dans la suppression thérapeutique de l'apoptose et de l'activité inflammatoire des neutrophiles.

2. Dérivés de riboside de 6-(benzylamino substitué) purine 2-substituée de formule générale I pour l'utilisation dans la suppression thérapeutique de l'apoptose et de l'activité inflammatoire des neutrophiles selon la revendication 1, dans lesquels les dérivés de formule générale I sont choisis parmi un groupe comprenant 2-(amino, chlore, fluor, mercapto, méthyle, C₁-C₆-alkylmercapto, C₁-C₆-alkylamino, hydroxy(C₁-C₆)alkylamino, amino(C₁-C₆)alkylamino)-6-(2-hydroxy-3-méthoxybenzylamino)purine riboside, 2-(amino, chlore, fluor, mercapto, méthyle, C₁-C₆-alkylmercapto, C₁-C₆-alkylamino, hydroxy(C₁-C₆)alkylamino, amino(C₁-C₆)alkylamino)-6-(2-hydroxy-4-méthoxybenzylamino)purine riboside, 2-(amino, chlore, fluor, mercapto, méthyle, C₁-C₆-alkylmercapto, C₁-C₆-alkylamino, hydroxy(C₁-C₆)alkylamino, amino(C₁-C₆)alkylamino)-6-(2-hydroxy-5-méthoxybenzylamino)purine riboside, 2-(amino, chlore, fluor, mercapto, méthyle, C₁-C₆-alkylmercapto, C₁-C₆-alkylamino, hydroxy(C₁-C₆)alkylamino, amino(C₁-C₆)alkylamino)-6-(2-hydroxy-6-méthoxybenzylamino)purine riboside, 2-(amino, chlore, fluor, mercapto, C₁-C₆-alkylmercapto, C₁-C₆-alkylamino, hydroxy(C₁-C₆)alkylamino, amino(C₁-C₆)alkylamino)-6-(2,3-dihydroxy-4-méthoxybenzylamino)purine riboside, 2-(amino, chlore, fluor, méthyle, mercapto, C₁-C₆-alkylmercapto, C₁-C₆-alkylamino, hydroxy(C₁-C₆)alkylamino, amino(C₁-C₆)alkylamino)-6-(2,5-dihydroxy-4-méthoxybenzylamino)purine riboside, 2-(amino, chlore, fluor, méthyle, mercapto, C₁-C₆-alkylmercapto, C₁-C₆-alkylamino, hydroxy(C₁-C₆)alkylamino, amino(C₁-C₆)alkylamino)-6-(2-hydroxy-3-méthylbenzylamino)purine riboside, 2-(amino, chlore, fluor, mercapto, méthyle, C₁-C₆-alkylmercapto, C₁-C₆-alkylamino, hydroxy(C₁-C₆)alkylamino, amino(C₁-C₆)alkylamino)-6-(2-hydroxy-4-méthylbenzylamino)purine riboside, 2-(amino, chlore, fluor, mercapto, méthyle, C₁-C₆-alkylmercapto, C₁-C₆-alkylamino, hydroxy(C₁-C₆)alkylamino, amino(C₁-C₆)alkylamino)-6-(2-hydroxy-5-méthylbenzylamino)purine riboside, 2-(amino, chlore, fluor, mercapto, méthyle, C₁-C₆-alkylmercapto, C₁-C₆-alkylamino, hydroxy(C₁-C₆)alkylamino, amino(C₁-C₆)alkylamino)-6-(2-hydroxy-6-méthylbenzylamino)purine riboside, 2-(amino, chlore, fluor, mercapto, méthyle, C₁-C₆-alkylmercapto, C₁-C₆-alkylamino, hydroxy(C₁-C₆)alkylamino, amino(C₁-C₆)alkylamino)-6-(2-hydroxy-3-chlorobenzylamino)purine riboside, 2-(amino, chlore, fluor, mercapto, méthyle, C₁-C₆-alkylmercapto, C₁-C₆-alkylamino, hydroxy(C₁-C₆)alkylamino, amino(C₁-C₆)alkylamino)-6-(2-hydroxy-4-chlorobenzylamino)purine riboside, 2-(amino, chlore, fluor, mercapto, méthyle, C₁-C₆-alkylmercapto, C₁-C₆-alkylamino, hydroxy(C₁-C₆)alkylamino, amino(C₁-C₆)alkylamino)-6-(2-hydroxy-5-chlorobenzylamino)purine riboside, 2-(amino, chlore, fluor, mercapto, méthyle, C₁-C₆-alkylmercapto, C₁-C₆-alkylamino, hydroxy(C₁-C₆)alkylamino, amino(C₁-C₆)alkylamino)-6-(2-hydroxy-6-chlorobenzylamino)purine riboside, 2-(amino, chlore, fluor, mercapto, méthyle, C₁-C₆-alkylmercapto, C₁-C₆-alkylamino, hydroxy(C₁-C₆)alkylamino, amino(C₁-C₆)alkylamino)-6-(2-hydroxy-3-fluorobenzylamino)purine riboside, 2-(amino, chlore, fluor, mercapto, méthyle, C₁-C₆-alkylmercapto, C₁-C₆-alkylamino, hydroxy(C₁-C₆)alkylamino, amino(C₁-C₆)alkylamino)-6-(2-hydroxy-5-fluorobenzylamino)purine riboside, 2-(amino, chlore, fluor, mercapto, méthyle, C₁-C₆-alkylmercapto, C₁-C₆-alkylamino, hydroxy(C₁-C₆)alkylamino, amino(C₁-C₆)alkylamino)-6-(2-hydroxy-3-brombenzylamino)purine riboside, 2-(amino, chlore, fluor, mercapto, méthyle, C₁-C₆-alkylmercapto, C₁-C₆-alkylamino, hydroxy(C₁-C₆)alkylamino, amino(C₁-C₆)alkylamino)-6-(2-hydroxy-3-iodobenzylamino)purine riboside, 2-(amino, chlore, fluor, mercapto, méthyle, C₁-C₆-alkylmercapto, C₁-C₆-alkylamino, hydroxy(C₁-C₆)alkylamino, amino(C₁-C₆)alkylamino)-6-(2,3-dihydroxy-4-méthylbenzylamino)purine riboside, 2-(amino, chlore, fluor, mercapto, méthyle, C₁-C₆-alkylmercapto, C₁-C₆-alkylamino, hydroxy(C₁-C₆)alkylamino, amino(C₁-C₆)alkylamino)-6-(2,5-dihydroxy-4-méthylbenzylamino)purine riboside, 2-(amino, chlore, fluor, mercapto, méthyle, C₁-C₆-alkylmercapto, C₁-C₆-alkylamino, hydroxy(C₁-C₆)alkylamino, amino(C₁-C₆)alkylamino)-6-(2,3-dihydroxy-4-chlorobenzylamino)purine riboside, 2-(amino, chlore, fluor, mercapto, méthyle, C₁-C₆-alkylmercapto, C₁-C₆-alkylamino, hydroxy(C₁-C₆)alkylamino, amino(C₁-C₆)alkylamino)-6-(2,5-dihydroxy-4-chlorobenzylamino)purine riboside, 2-(amino, chlore, fluor, mercapto, méthyle, C₁-C₆-alkylmercapto, C₁-C₆-alkylamino, hydroxy(C₁-C₆)alkylamino, amino(C₁-C₆)alkylamino)-6-(2,6-dihydroxy-4-chlorobenzylamino)purine riboside, 2-(amino, chlore, fluor, mercapto, méthyle, C₁-C₆-alkylmercapto, C₁-C₆-alkylamino, hydroxy(C₁-C₆)alkylamino, amino(C₁-C₆)alkylamino)-6-(2-hydroxy-3-aminobenzylamino)purine riboside, 2-(amino, chlore, fluor, mercapto, méthyle, C₁-C₆-alkylmercapto, C₁-C₆-alkylamino, hydroxy(C₁-C₆)alkylamino, amino(C₁-C₆)alkylamino)-6-(2-hydroxy-4-aminobenzylamino)purine riboside, 2-(amino, chlore, fluor, méthyle, mercapto, C₁-C₆-alkylmercapto, C₁-C₆-alkylamino, hydroxy(C₁-C₆)alkylamino, amino(C₁-C₆)alkylamino)-6-(2-hydroxy-5-aminobenzylamino)purine riboside, 2-(amino, chlore, méthyle, mercapto, C₁-C₆-alkylmercapto, C₁-C₆-alkylamino, hydroxy(C₁-C₆)alkylamino, amino(C₁-C₆)alkylamino)-6-(2-hydroxy-5-mercaptobenzylamino)purine riboside, 2-(amino, chlore, fluor, méthyle, mercapto, C₁-C₆-alkylmercapto, C₁-C₆-alkylamino, hydroxy(C₁-C₆)alkylamino, amino(C₁-C₆)alkylamino)-6-(2-amino-3-méthoxybenzylamino)purine riboside, 2-(amino, chlore, fluor, méthyle, mercapto, C₁-C₆-alkylmercapto, C₁-C₆-alkylamino, hydroxy(C₁-C₆)alkylamino, amino(C₁-C₆)alkylamino)-6-(2-amino-4-méthoxybenzylamino)purine riboside, 2-(amino, chlore, fluor, méthyle, mercapto, C₁-C₆-alkylmercapto, C₁-C₆-alkylamino, hydroxy(C₁-C₆)alkylamino, amino(C₁-C₆)alkylamino)-6-(2-amino-5-méthoxybenzylamino)purine riboside, 2-(amino, chlore, fluor, méthyle, mercapto, C₁-C₆-alkylmercapto, C₁-C₆-alkylamino, hydroxy(C₁-C₆)alkylamino, amino(C₁-C₆)alkylamino)-6-(2-amino-3-méthylbenzylamino)purine riboside, 2-(amino, chlore, fluor, méthyle, mercapto, C₁-C₆-alkylmercapto, C₁-C₆-alkylamino, hydroxy(C₁-C₆)alkylamino, amino(C₁-C₆)alkylamino)-6-(2-amino-5-méthylbenzylamino)purine riboside, 2-(amino, chlore, fluor, méthyle, mercapto, C₁-C₆-alkylmercapto)-6-(2-amino-3-chlorobenzylamino)purine riboside, 2-(amino, chlore, fluor, méthyle, mercapto, C₁-C₆-alkylmercapto, C₁-C₆-alkylamino, hydroxy(C₁-C₆)alkylamino, amino(C₁-C₆)alkylamino)-6-(2-amino-4-chlorobenzylamino)purine riboside, 2-(amino, chlore, fluor, méthyle, mercapto, C₁-C₆-alkylmercapto, C₁-C₆-alkylamino, hydroxy(C₁-C₆)alkylamino, amino(C₁-C₆)alkylamino)-6-(2-amino-5-chlorobenzylamino)purine riboside, 2-(amino, chlore, fluor, méthyle, mercapto, C₁-C₆-alkylmercapto, C₁-C₆-alkylamino, hydroxy(C₁-C₆)alkylamino, amino(C₁-C₆)alkylamino)-6-(2-amino-6-chlorobenzylamino)purine riboside, 2-(amino, chlore, fluor, méthyle, mercapto, C₁-C₆-alkylmercapto, C₁-C₆-alkylamino, hydroxy(C₁-C₆)alkylamino, amino(C₁-C₆)alkylamino)-6-(2-amino-3-fluorobenzylamino)purine riboside, 2-(amino, chlore, fluor, méthyle, mercapto, C₁-C₆-alkylmercapto, C₁-C₆-alkylamino, hydroxy(C₁-C₆)alkylamino, amino(C₁-C₆)alkylamino)-6-(2-amino-5-fluorobenzylamino)purine riboside, 2-(amino, chlore, fluor, méthyle, mercapto, C₁-C₆-alkylmercapto, C₁-C₆-alkylamino, hydroxy(C₁-C₆)alkylamino, amino(C₁-C₆)alkylamino)-6-(2-amino-3-brombenzylamino)purine riboside, 2-(amino, chlore, fluor, méthyle, mercapto, C₁-C₆-alkylmercapto, C₁-C₆-alkylamino, hydroxy(C₁-C₆)alkylamino, amino(C₁-C₆)alkylamino)-6-(2,3-diaminobenzylamino)purine riboside, 2-(amino, chlore, fluor, méthyle, mercapto, C₁-C₆-alkylmercapto, C₁-C₆-alkylamino, hydroxy(C₁-C₆)alkylamino, amino(C₁-C₆)alkylamino)-6-(2,5-diaminobenzylamino)purine riboside, 2-(amino, chlore, fluor, méthyle, mercapto, C₁-C₆-alkylmercapto, C₁-C₆-alkylamino, hydroxy(C₁-C₆)alkylamino, amino(C₁-C₆)alkylamino)-6-(2,6-diamino-3-chlorobenzylamino)purine riboside, 2-(amino, chlore, fluor, méthyle, mercapto, C₁-C₆-alkylmercapto, C₁-C₆-alkylamino, hydroxy(C₁-C₆)alkylamino, amino(C₁-C₆)alkylamino)-6-(2,6-diamino-4-chlorobenzylamino)purine riboside, 2-(amino, chlore, fluor, méthyle, mercapto, C₁-C₆-alkylmercapto, C₁-C₆-alkylamino, hydroxy(C₁-C₆)alkylamino, amino(C₁-C₆)alkylamino)-6-(2,3-diamino-4-chlorobenzylamino)purine riboside,
et leurs sels pharmaceutiquement acceptables avec les métaux alcalins, l'ammoniaque ou les amines, ou leurs sels d'addition avec les acides.

3. Dérivés de riboside de 6-(benzylamino substitué) purine 2-substituée de formule générale I pour l'utilisation dans la suppression thérapeutique de l'apoptose et de l'activité inflammatoire des neutrophiles selon la revendication 1, dans lesquels les dérivés de formule générale I sont choisis parmi un groupe comprenant 2-(chlore, fluor, C₁-C₆-alkylmercapto, C₁-C₆-alkylamino, hydroxy(C₁-C₆)alkylamino, amino(C₁-C₆)alkylamino)-6-(2-hydroxy-3-méthoxybenzylamino)purine riboside, 2-(chlore, fluor, C₁-C₆-alkylmercapto, C₁-C₆-alkylamino, hydroxy(C₁-C₆)alkylamino, amino(C₁-C₆)alkylamino)-6-(2-hydroxy-5-méthoxybenzylamino)purine riboside, 2-(chlore, fluor, C₁-C₆-alkylmercapto, C₁-C₆-alkylamino, hydroxy(C₁-C₆)alkylamino, amino(C₁-C₆)alkylamino)-6-(2-hydroxy-3-méthylbenzylamino)purine riboside, 2-(chlore, fluor, C₁-C₆-alkylmercapto, C₁-C₆-alkylamino, hydroxy(C₁-C₆)alkylamino, amino(C₁-C₆)alkylamino)-6-(2-hydroxy-5-méthylbenzylamino)purine riboside, 2-(chlore, fluor, C₁-C₆-alkylmercapto, C₁-C₆-alkylamino, hydroxy(C₁-C₆)alkylamino, amino(C₁-C₆)alkylamino)-6-(2-hydroxy-3-chlorobenzylamino)purine riboside, 2-(chlore, fluor, C₁-C₆-alkylmercapto, C₁-C₆-alkylamino, hydroxy(C₁-C₆)alkylamino, amino(C₁-C₆)alkylamino) -6-(2-hydroxy-5-chlorobenzylamino)purine riboside, 2-(chlore, fluor, C₁-C₆-alkylmercapto, C₁-C₆-alkylamino, hydroxy(C₁-C₆)alkylamino, amino(C₁-C₆)alkylamino)-6-(2-hydroxy-5-fluorobenzylamino)purine riboside, 2-(chlore, fluor, C₁-C₆-alkylmercapto, C₁-C₆-alkylamino, hydroxy(C₁-C₆)alkylamino, amino(C₁-C₆)alkylamino)-6-(2-amino-5-chlorobenzylamino)purine riboside, 2-(chlore, fluor, C₁-C₆-alkylmercapto, C₁-C₆-alkylamino, hydroxy(C₁-C₆)alkylamino, amino(C₁-C₆)alkylamino)-6-(2-amino-5-fluorobenzylamino)purine riboside, 2-(chlore, fluor, C₁-C₆-alkylmercapto, C₁-C₆-alkylamino, hydroxy(C₁-C₆)alkylamino, amino(C₁-C₆)alkylamino)-6-(2-hydroxy-5-aminobenzylamino)purine riboside,
et leurs sels pharmaceutiquement acceptables avec les métaux alcalins, l'ammoniaque ou les amines, ou leurs sels d'addition avec les acides.

4. Dérivés de riboside de 6-(benzylamino substitué) purine 2-substituée de formule générale I selon l'une quelconque des revendications 1 à 3 pour l'utilisation dans le traitement ou dans la prophylaxie des maladies inflammatoires.

5. Dérivés de riboside de 6-(benzylamino substitué) purine 2-substituée de formule générale I selon l'une quelconque des revendications 1 à 3 pour l'utilisation dans le traitement d'une maladie choisie parmi un groupe comprenant la vasculite associée à ANCA, l'insuffisance rénale progressive induite par la glomérulonéphrite (ESRF), la neutropénie, l'emphysème, la fièvre méditerranéenne familiale (FMF), l'arthralgie, la péritonite, l'amyloïdose, les maladies inflammatoires chroniques.

6. Utilisation des dérivés de riboside de 6-(benzylamino substitué) purine 2-substituée de formule générale I selon l'une quelconque des revendications 1 à 3 en tant que facteurs anti-apoptotiques des cellules végétales et animales dans les cultures de tissus.
